(19) Europäisches Patentamt
European Patent Office
Office européen des brevets

(11) Publication number: **0 572 437 B1**

# (12) EUROPEAN PATENT SPECIFICATION

(45) Date of publication of patent specification: **26.04.95**

(51) Int. Cl.[6]: **C07D 239/95**, C07D 401/04, C07D 401/14, C07D 403/12, A61K 31/505

(21) Application number: **92903632.5**

(22) Date of filing: **08.01.92**

(86) International application number: **PCT/US92/00028**

(87) International publication number: **WO 92/14716 (03.09.92 92/23)**

## (54) 2,4-DIAMINOOUINAZOLINES DERIVATIVES FOR ENHANCING ANTITUMOR ACTIVITY.

(30) Priority: **20.02.91 US 657922**

(43) Date of publication of application: **08.12.93 Bulletin 93/49**

(45) Publication of the grant of the patent: **26.04.95 Bulletin 95/17**

(84) Designated Contracting States: **AT BE CH DE DK ES FR GB GR IT LI LU NL SE**

(56) References cited:
**EP-A- 0 322 133**
**US-A- 3 663 706**

**JOURNAL OF MEDICINAL CHEMISTRY. vol. 28, no. 1, 1985, WASHINGTON US pages 12 -17; J. MILLEN ET AL.: '2-(BETA-ARYLETHYLAMINO)- AND 4-(beta-ARYLETHYLAMINO)OUINAZOLINES as PHOSPHODIESTERASE INHIBITORS' cited in theapplication**

(73) Proprietor: **PFIZER INC.**
**235 East 42nd Street**
**New York, N.Y. 10017 (US)**

(72) Inventor: **COE, Jotham, W.**
**151 Lamphere Road**
**Mystic, CT 06355 (US)**
Inventor: **FLIRI, Anton, F., J.**
**596 West Thames Street**
**Norwich, CT 06360 (US)**
Inventor: **KANEKO, Takushi**
**393 Northwood Drive**
**Guilford, CT 06437 (US)**
Inventor: **LARSON, Eric, R.**
**162 Lantern Hill Road**
**Mystic, CT 06355 (US)**

(74) Representative: **Wood, David John**
**PFIZER LIMITED,**
**Ramsgate Road**
**Sandwich,**
**Kent CT13 9NJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

Background of the Invention

This invention relates to 2,4-diaminoquinazolines and their use as sensitizers of tumor cells to anticancer agents.

In cancer chemotherapy the effectiveness of anticancer drugs is often limited by the resistance of tumor cells. Some tumors such as of the colon, pancreas, kidney and liver are generally innately resistant, and other responding tumors often develop resistance during the course of chemotherapy. The phenomena of multidrug resistance (MDR) is characterized by the tumor cell's cross-resistance to structurally unrelated drugs. The drugs which are the target of resistance include adriamycin, daunomycin, vinblastine, vincristine, actinomycin D and etoposide. The resistance cells are often associated with over-expression of the mdrl gene. This gene product is a family of 140-220 kd trans-membrane phosphoglycoprotein (P-glycoprotein) which functions as an ATP-dependent efflux pump. Thus, it has been postulated that this efflux mechanism keeps the intracellular level of the anticancer drug low, allowing the tumor cells to survive.

In recent years various substances such as verapamil, nifedipine and diltiazem have been used in in vitro experimental systems to reverse the MDR phenomena. More recently some of these agents have been tested clinically as MDR reversing agents. Little efficacy has been observed with verapamil or trifluoroperazine. Thus, there is a need for an effective MDR reversing agent.

The 2,4-diaminoquinazolines are prepared by known methods utilizing 2,4-dichloroquinazolines [Postovskii and Goncharova, Zh. Obshch. Khim., 32, 3323 (1962)]. Curd et al. (J. Chem. Soc., 1947, 775) reported the synthesis of 2,4-dichloroquinazolines from the corresponding 2,4(1H, 3H)quinazolinedione. The Wellcome Foundation discloses 2,4-diaminoquinazolines of general structure D as antibacterials [GB patent 806772 (1958)]. Hess [US 3,511,836 (1970)] patented compounds of structures E, F, and G as antihypertensive agents. Wijbe [GB patent 1,390,014 (1975)] patented a process for compounds of structure H and these compounds are claimed to be antibacterials. Lacefield [US patent 3,956,495 (1976)) describes compounds of the general formula I as antithrombotic agents. Crenshaw [US patent 4,098,788 (1978)] patented a process for the production of compounds of formula J. Hess [European Patent 0,028,473 (1981)] describes chloro- and alkoxy-substituted 2,4-diaminoquinazolines of formula K. Ife et al. describe compounds of general structure L as inhibitors of gastric acid secretion [WO 89/0527 (1989)]. Compounds of structures M and N were published as phosphodiesterase inhibitors [Miller, J. Med. Chem., 28, 12 (1985)]. Richter et al. published compounds of structure O as inhibitors of dihydrofolate reductase [J. Med. Chem., 17, 943 (1974)]. In search of compounds with herbicidal activity Miki et al. reported the synthesis of 2,4-dialkylaminoquinazolines (P) (Chem. Pharm. Bull. 30, 2313 (1982)]. Arylazidoprazosin (Q) has been shown to bind to P-glycoprotein [Safa et al., Biochem. Biophys. Res. Comm. 166, 259 (1990)].

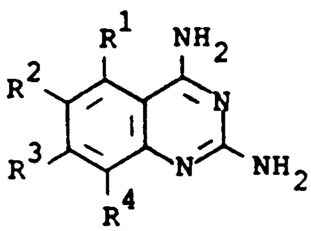

D

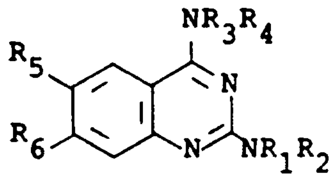

E

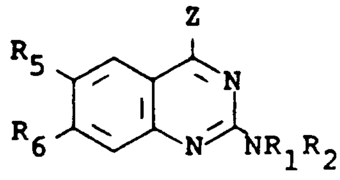

F

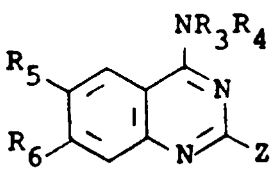

G

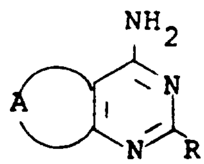

H

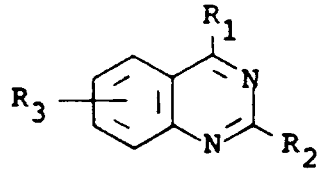

I

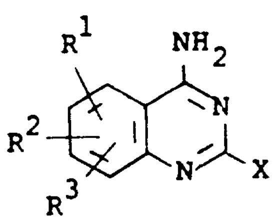

J

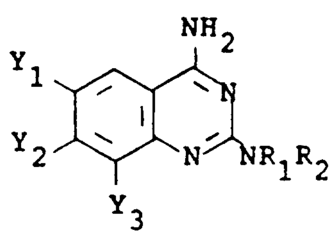

K

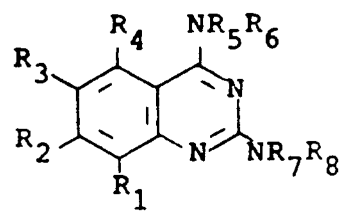

L

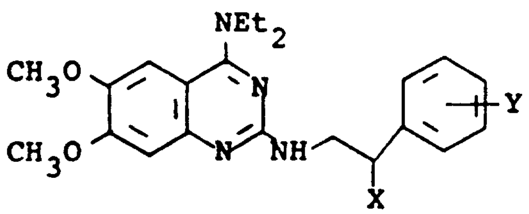

M

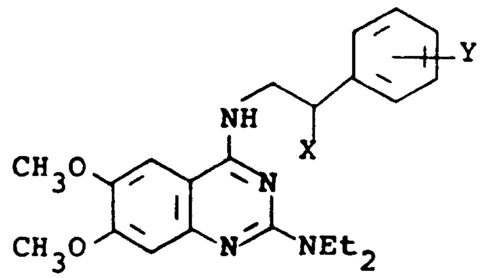

N

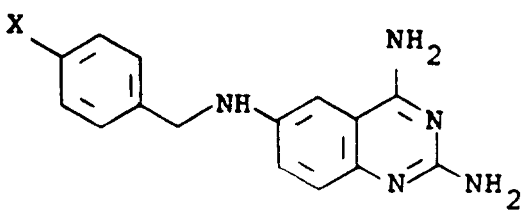

O

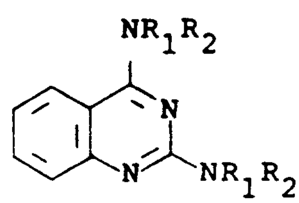

P

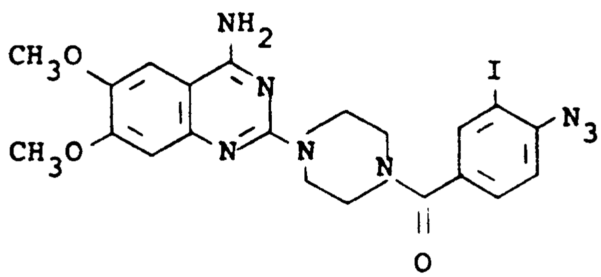

Q

Summary of the Invention

The compound of the present invention are of the formula

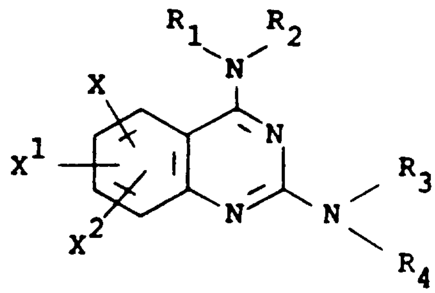

I

or a pharmaceutically acceptable acid addition salt thereof wherein X is alkyl of one to three carbon atoms, alkoxy of one to three carbon atoms, chloro, fluoro, amino, alkylamino of one to three carbon atoms, dialkylamino of two to six carbon atoms or trifluoromethyl; $X^1$ is hydrogen, alkyl of one to three carbon atoms, alkoxy of one to three carbon atoms, fluoro, chloro, dialkylamino of two to six carbon atoms; $X^2$ is hydrogen, alkyl of one to three carbon atoms or alkoxy of one to three carbon atoms; X and $X^1$ when taken together are ethylenedioxy or methylenedioxy; $R_1$ is hydrogen or alkyl of one to three carbon atoms; $R_2$ is alkyl of one to ten carbon atoms or aralkyl of the formula

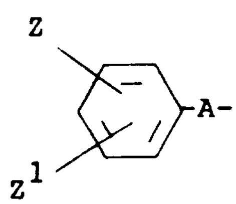

where Z and $Z^1$ are each hydrogen, alkyl of one to three carbon atoms, alkoxy of one to three carbon atoms, fluoro, chloro, bromo, trifluoromethyl or dialkylamino of two to six carbon atoms, A is a chemical bond or alkylene of one to four carbon atoms and Z and $Z^1$ when taken together are ethylenedioxy or methylenedioxy; $R_3$ is hydrogen or alkyl of one to three carbon atoms; $R_4$ is aralkyl of the formula

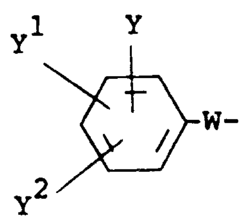

where W is alkylene of one to four carbon atoms, Y and $Y^1$ are each hydrogen, alkyl of one to three carbon atoms, alkoxy of one to three carbon atoms, fluoro, chloro, bromo, trifluoromethyl or dialkylamino of two to six carbon atoms, $Y^2$ is hydrogen, alkyl of one to three carbon atoms or alkoxy of one to three carbon atoms and $Y^1$ and $Y^2$ when taken together are ethylenedioxy or methylenedioxy; and $R_3$ and $R_4$ when taken together with the nitrogen to which they are attached are 4-phenylpiperazino, 4-alkoxyethoxypiperidino said alkoxy having from one to three carbon atoms or 4-alkoxycarbonylpiperazino said alkoxy having from one to three carbon atoms with the proviso that when $R_1$ is alkyl having one to three carbons then $R_2$ is not alkyl having one to eight carbons; when $X^1$ and $X^2$ are each hydrogen, X is amino, alkylamino of one to three carbon atoms, dialkylamino of two to six carbon atoms or trifluoromethyl;
when $X^1$ is H and $X^2$ is methoxy or $X^1$ is methoxy and $X^2$ is hydrogen, X is methoxy, $R_1$ is ethyl, $R_3$ is hydrogen, $R_4$ is the aralkyl where W is ethylene and Y, $Y^1$ and $Y^2$ are each hydrogen, then $R_2$ is not ethyl;

and
when $R_1$ is alkyl having one to three carbons and $R_2$ is a moiety of the formula

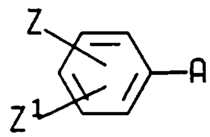

where A is $-CH_2-$, then Z and $Z^1$ cannot both be hydrogen.

A preferred group of compounds are those wherein X and $X^1$ are each methoxy, $R_1$ is methyl, $R_2$ is aralkyl of the formula

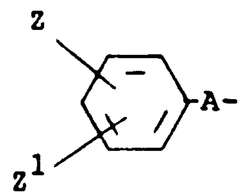

where Z is hydrogen, $R_3$ is hydrogen and $R_4$ is aralkyl of the formula

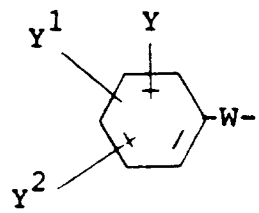

where W is $-(CH_2)_2-$, Y and $Y^1$ are each methoxy and $Y^2$ is hydrogen. Especially preferred within this group are the compounds where X is 6-methoxy, $X^1$ is 7-methoxy, $X^2$ is hydrogen, $Z^1$ is 2-methoxy, A is $-CH_2-$, Y is 3-methoxy and $Y^1$ is 4-methoxy, where X is 6-methoxy, $X^1$ is 7-methoxy, $X^2$ is hydrogen, $Z^1$ is hydrogen, A is $-CH_2-$, Y is 3-methoxy and $Y^1$ is 4-methoxy, where X is 6-methoxy, $X^1$ is 7-methoxy, $X^2$ is 8-methoxy, $Z^1$ is hydrogen, A is $-CH_2-$, Y is 3-methoxy and $Y^1$ is 4-methoxy, where X is 6-methoxy, $X^1$ is 7-methoxy, $X^2$ is hydrogen, $Z^1$ is 4-fluoro, A is $-CH_2-$, Y is 3-methoxy and $Y^1$ is 4-methoxy.

A second group of preferred compounds are those where $X^2$ is hydrogen, $R_1$ is methyl, $R_2$ is aralkyl of the formula

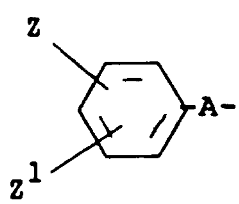

$R^3$ is hydrogen and $R_4$ is aralkyl of the formula

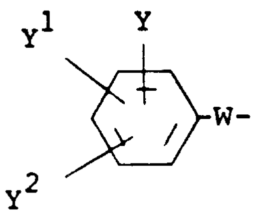

where W is $-(CH_2)_2-$, Y and $Y^1$ are each methoxy and $Y^2$ is hydrogen. Especially preferred within this group are the compounds where X is 6-methoxy, $X^1$ is 7-methoxy, Z is 3-methoxy, $Z^1$ is 4-methoxy, A is a chemical bond, Y is 2-methoxy and $Y^1$ is 3-methoxy, where X and $X^1$ are methylenedioxy, Z and $Z^1$ are each hydrogen, A is $-CH_2-$, Y is 3-methoxy and $Y^1$ is 4-methoxy and where X is 6-methoxy, $X^1$ is 7-methoxy, Z is 2-methoxy, $Z^1$ is 3-methoxy, A is $-CH_2$, Y is 3-methoxy and $Y^1$ is 4-methoxy.

The present invention also includes a method of inhibiting a P-glycoprotein in a mammal in need of such treatment which comprises administering to said mammal a P-glycoprotein inhibiting amount of a compound of formula I. Preferred is the method where the mammal is a human suffering from cancer and said compound is administered with an anticancer effective amount of a chemotherapeutic agent.

Also included is a pharmaceutical composition for administration to a mammal which comprises a P-glycoprotein inhibiting amount of a compound of formula I, a pharmaceutically acceptable carrier and, optionally, an anticancer effective amount of a chemotherapeutic agent.

As previously indicated, the compounds of formula I form pharmaceutically acceptable acid addition salts. Said pharmaceutically acceptable acid addition salts include, but are not limited to, those with HCl, HBr, $HNO_3$, $H_2SO_4$, $H_3PO_4$, $CH_3SO_3H$, p-$CH_3C_6H_4SO_3H$, $CH_3CO_2H$, gluconic acid, tartaric acid, maleic acid and succinic acid. In the case of those compounds of the formula (I) which contain a further basic nitrogen, it will, of course, be possible to form diacid addition salts (e.g., the dihydrochloride) as well as the usual monoacid addition salt.

As one skilled in the art recognized, compounds of formula I have the potential for containing asymmetric carbon atoms. All these potential isomers are considered within the scope of the present invention.

## Detailed Description of the Invention

Compounds of the present invention are prepared with the reaction of a 2,4-dichloroquinazoline with an equivalent of an appropriate amine, $R_1R_2NH$, followed by the reaction of the product, a 2-chloro-4-aminoquinazoline derivative, with a second equivalent of an appropriate amine, $R_3R_4NH$.

In a more detailed description of the procedure, one molar equivalent of an optionally substituted 2,4-dichloroquinazoline and one molar equivalent of a tertiary amine-acid scavenger, such as triethylamine, N-methylmorpholine or diethylisopropylamine and one molar equivalent of an amine, $R_1R_2NH$, are combined in an anhydrous solvent such as dimethylacetamide, dioxane, chloroform, or N-methyl-2-pyrrolidone and maintained at from 0 °C to about 25 °C for a period of 1 to 48 hours.

The reaction mixture can be filtered and the filtrate concentrated to dryness in vacuo, or the reaction mixture can be quenched in water and the intermediate product either filtered or extracted with a water immiscible solvent such as methylene chloride or ethyl acetate. Removal of the extracting solvent provides the desired product. Frequently, the residue can be induced to crystallize by trituration with an organic solvent, and further purified by recrystallization or column chromatography.

The second step of the sequence leading to the products of the present invention consists of combining one molar equivalent of the appropriate 2-chloro-4-aminoquinazoline with either two molar equivalents of an amine, $R_3R_4NH$, or one equivalent of said amine and one equivalent of a tertiary amine-acid scavenger as described above in a reaction-inert solvent such as ethoxyethoxyethanol, butanol, amyl alcohol or cyclohexanol for a period of 5 minutes to several hours at reaction temperatures of 100-200 °C.

The reaction mixture can be cooled to room temperature and treated with a 1-N solution of an appropriate acid, such as hydrochloric acid to give a precipitate of the desired product as the hydrochloride salt. Other acids would give the corresponding acid addition salt. In instances where the acid addition salt does not precipitate the free base product can be isolated by chromatography of the crude material on silica gel using an eluant such as chloroform, ethyl acetate, diethyl ether, methanol methylene chloride,

ethanol or mixtures thereof and subsequently converted to the acid addition salt product. The products are isolated by removing the eluting solvents in vacuo. Purification of the product can be done by recrystallization.

Generation of the free base from an acid addition salt can readily be carried out by treating an aqueous solution or suspension of the salt with at least one equivalent of an organic or inorganic base followed by extraction of the free base product with a water immiscible solvent such as ethyl acetate or methylene chloride. Removal of the solvent gives the desired base.

Compounds of formula I are inhibitors of the functions of P-glycoprotein, particularly human mdr 1 protein or P-glycoprotein related and membrane associate proteins which are participating in the transport of xenobiotics or proteins across membranes e.g., cell membranes of eukariotic and proeukariotic origin e.g., pmfdr, however not exclusive or restricted to these examples.

Compounds enclosed in general formula I are useful in combination chemotherapy of cancer, malaria, viral infections such as AIDS, in therapy of septic shock syndrome or inflammation and may be useful in enhancing the tissue penetration of drugs where the penetration of these xenobiotics is limited due to the presence of P-glycoprotein or P-glycoprotein related functional proteins. Compounds of formula I increase the activity/efficacy of adriamycin, daunomycin, etoposide, epipodophyllotoxin congoners, actinomycin D, emetin, vincristin, vinblastin, chloroquine, antracycline antibiotics and of drugs which are structurally and functionally related to the above mentioned examples, in particular when the activity of these drugs has been shown to be limited due to the presence and function of P-glycoprotein, e.g. human mdr 1 protein or P-glycoprotein related proteins.

The compounds of the present invention are evaluated as potentiators of chemotherapeutic agents using a Cellular Drug Retention Assay. This assay was designed to study the effect of compounds on cellular retention of radiolabeled drug. In this case 14C-adriamycin retention by multidrug resistant human carcinoma cells, KBVI, is measured.

KBVI cells are routinely grown in tissue culture as monolayers in DMEM high glucose medium containing 1 ug/ml vinblastine 10% heat inactivated fetal calf serum and supplemented with Glutamine, Pen-Strep and Garamycin.

The assay protocol (described below) should be applicable, with minor modifications, to a wide variety of cell lines grown in tissue culture.

Assay Protocol:

(1) Seed replicate 6-well tissue culture plates with 1.2 x 10E6 cells per 2 ml per well in absence of Vinblastine;

(2) Incubate 24 hrs at 37 degrees in humidified incubator (5% CO2);

(3) Aspirate off the spent media and overlay monolayers with 2 ml/well of fresh medium that is 2 uM in Adriamycin (2 uM unlabeled Adriamycin + 20000 cpm of 14C-Adr) and the test agent at concentrations varying from 0 to 100 uM;

(4) Following incubation for 3 hours at 37 degrees in humidified incubator, remove media and wash monolayers twice with 2 ml of ice-cold buffered saline;

(5) Detach monolayers using 0.5 ml of trypsin/EDTA, collect detached cells and transfer to scintillation vial. Rinse wells once with 0.5 ml of buffered saline and add to same vial containing cells;

(6) Add 5 ml of Beckman Ready-Safe scintillation fluid to vial, vortex and determine radioactivity per sample using a scintillation counter (10 minutes per sample);

(7) For background control: pre-incubate monolayers at 4 degrees for 15 minutes then remove media and add fresh ice-cold media containing Adr (see step 3). Following incubation for 3 hours at 4 degrees remove media and wash monolayers twice with 2 ml ice-cold buffered saline, then proceed as in step 5;

(8) Results are expressed as T/C and ED3x values as defined below:

T/C = pmoles Adr per 10E6 cells treated with test agent/ pmoles Adr per 10E6 untreated cells

ED3x = concentration of test agent that produces a 3 fold increase in cellular accumulation of radiolabeled Adr, i.e. T/C = 3.

Calculations:

Specific cpm = [sample cpm - background cpm]

Specific activity = [cpm/total conc. of Adr]

pmoles Adr = [specific cpm/specific activity]

pmoles Adr per 10E6 cells = [(pmoles Adr per well/number of cells per well) x 10E6 cells]

As previously mentioned compounds of the present invention and salts thereof are useful in potentiating the anticancer effects of chemotherapeutic agents. Such agents can include adriamycin, daunomycin, aclacinomycin A, actinomycin C, actinomycin D, mithramycin, toyomycin, vinblastine, maytansine, bruceantin, homoharintonin, anguindin, neocarcinostatin, mitomycin C and anthramycin.

The compounds of the present invention can be administered with, 24 hours before or up to 72 hours after the administration of the chemotherapeutic agents. When administered with said agents, they can be taken either separately or coadministered in the same formulation.

The compounds of the present invention whether taken separately or in combination with an anti-cancer agent, are generally administered in the form of pharmaceutical compositions comprising at least one of the compounds of formula I and optionally a chemotherapeutic agent, together with a pharmaceutically acceptable vehicle or diluent. Such compositions are generally formulated in a conventional manner utilizing solid or liquid vehicles or diluents as appropriate to the mode of desired administration: for oral administration, in the form of tablets, hard or soft gelatin capsules, suspensions, granules, powders and the like, and, for parenteral administration, in the form of injectable solutions or suspensions, and the like.

For use in the potentiation of anti-cancer agents in a mammal, including man, a compound of formula I is given in an amount of about 0.5-100 mg/kg/day, in single or divided doses. A more preferred dosage range is 2-50 mg/kg/day, although in particular cases, at the discretion of the attending physician, doses outside the broader range may be required. The preferred route of administration is generally oral, but parenteral administration (e.g. intramuscular, intravenous, intradermal) will be preferred in special cases, e.g., where oral absorption is impaired as by disease, or where the patient is unable to swallow.

The present invention is illustrated by the following examples, but is not limited to the details or scope thereof.

EXAMPLE 1

2-(3,4-Dimethoxyphenethylamino)-4-(N-methyl-benzylamino)-6,7-dimethoxyquinazoline hydrochloride (X = 6-$CH_3O$; $X^1$ = 7-$CH_3O$; $X^2$ = H; $R_1$ = $CH_3$; $R_2$ = $C_6H_5CH_2$-; $R_3$ = H; and $R_4$ = 3,4-$(CH_3O)_2C_6H_3(CH_2)_2$-)

A. 2-chloro-4-(N-methyl-benzylamino)-6,7-dimethylquinazoline

To a solution of 26 g of 2,4-dichloro-6,7-dimethoxyquinazoline and 10 g of triethylamine in 350 ml of dry dimethylacetamide was added 12 g of N-methylbenzylamine. The reaction mixture was stirred at room temperature for 5 hrs and was then diluted with 1000 ml of water. The precipitated product was filtered, washed with water (1 x 200 ml) and suspended in 200 ml of hot ethanol. A sample was recrystallized from methanol, m.p. 187-188 °C.

B. 2-(3,4-dimethoxyphenethylamino)-4-(N-methylbenzylamino)-6,7-dimethoxyquinazoline hydrochloride

A mixture of 1.03 g of the product from Example 1A, 543 mg of 3,4-dimethoxyphenethylamine and 387 mg of diisopropylethylamine in 2 g of ethoxyethoxyethanol was heated with stirring for 2 hrs under a nitrogen atmosphere. The reaction mixture was cooled, diluted with a small amount of chloroform and applied to a column of silica gel (30 g). The column was eluted with chloroform and then 2% methanol in chloroform (V:V). The fractions containing the product ware combined and concentrated in vacuo to a yellow-residue. The residue was dissolved in 1N hydrochloric acid in methanol and the resulting precipitate filtered and dried, 550 mg, m.p. 201-202 °C, $M^+$ = 489.2.

EXAMPLES 2-37

Employing the procedure of Example 1A and B and starting with the appropriate reagents, the following compounds were prepared:

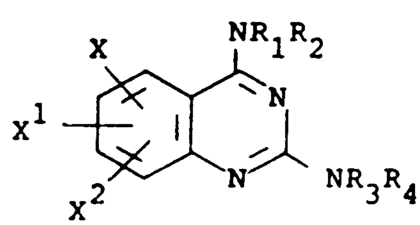

Example 2: X = 6-$CH_3O$; $X^1$ = 7-$CH_3O$; $X^2$ = H; $R_1$ = $CH_3$; $R_2$ = 2-$CH_3OC_6H_4CH_2$-; $R_3$ = H and $R_4$ = 3,4-$(CH_3O)_2)C_6H_3$-$(CH_2)_2$-; m.p. 204.5-206 °C, $M^+$ = 519.2.

Example 3: X = 6-$CH_3O$; $X^1$ = 7-$CH_3O$; $X^2$ = H; $R_1$ = H; $R_2$ = 3,4-$(CH_3O)_2C_6H_3$-; $R_3$ = H; and $R_4$ = 3,4-$(CH_3O)_2$-$C_6H_3(CH_2)_2$-; m.p. 231-233 °C, $M^+$ 521.0.

Example 4: X = 6-$CH_3O$; $X^1$ = 7-$CH_3O$; $X^2$ = H; $R_1$ = H; $R_2$ = 3,4-$(CH_3O)_2C_6H_3$-; $R_3$ = H; and $R_4$ = 2,3-$(CH_3O)_2$-$C_6H_3(CH_2)_2$-; m.p. 236-238 °C, $M^+$ = 521.0.

Example 5: X = 6-$CH_3O$; $X^1$ = 7-$CH_3O$; $X^2$ = H; $R_1$ = $CH_3$; $R_2$ = 3,4-$(CH_3O)_2C_6H_3$-; $R_3$ = H; and $R_4$ = 3,4$(CH_3O)_2C_6H_3$-$(CH_2)_2$-; m.p. 250 °C, $M^+$ 475.2.

Example 6: X, $X^1$ and $X^2$ = H; $R_1$ = H; $R_2$ = $C_6H_5$-; $R_3$ = H; and $R_4$ = 2,3-$(CH_3O)_2C_6H_3(CH_2)_2$-; m.p. 231.5-232.5 °C, $M^+$ 401.0.

Example 7: X = 6-$CH_3O$; $X^1$ = 7-$CH_3O$; $X^2$ = H; $R_1$ = $CH_3$; $R_2$ = 3,4-$(CH_3O)_2C_6H_3$-; $R_3$ = H; and $R_4$ = 2,3-$(CH_3O)_2$-$C_6H_3(CH_2)_2$-; m.p. 212-213 °C, $M^+$ 475.2.

Example 8: X = 6-$CH_3O$; $X^1$ = 7-$CH_3O$; $X^2$ = H; $R_1$ = H; $R_2$ = $C_6H_5$-; $R_3$ = H; and $R_4$ = 3,4-$(CH_3O)$-$_2C_6H_3(CH_2)_2$-; m.p. 109-112 °C (free base), $M^+$ 460.5.

Example 9: X = 6-$CH_3O$; $X^1$ = 7-$CH_3O$; $X^2$ = H; $R_1$ = $CH_3$; $R_2$ = 3-Cl-4-$FC_6H_3CH_2$-; $R_3$ = H; and $R_4$ = 3,4-$(CH_3O)_2$-$C_6H_3(CH_2)_2$-; m.p. 129-131 °C, $M^+$ 541.2.

Example 10: X = 6-$CH_3O$; $X^1$ = 7-$CH_3O$; $X^2$ = H; $R_1$ = $CH_3$; $R_2$ = 2,6-$(CH_3O)_2C_6H_3CH_2$-; $R_3$ = H; and $R_4$ = 3,4-$(CH_3O)_2$-$C_6H_3(CH_2)_2$-; m.p. 177-179 °C, $M^+$ 549.3.

Example 11: X = 6-$CH_3O$; $X^1$ = 7-$CH_3O$; $X^2$ = H; $R_1$ = H; $R_2$ = $C_6H_5CH_2$; $R_3$ = H; and $R_4$ = 3,4-$(CH_3O)_2C_6H_3(CH_2)_2$-; m.p. 249-251 °C, $M^+$ 474.5.

Example 12: X = 6-$CH_3O$; $X^1$ = 7-$CH_3O$; $X^2$ = H; $R_1$ = H; $R_2$ = $C_6H_5CH_2$-; $R_3$ = H; and $R_4$ = 2,3-$(CH_3O)_2C_6H_3(CH_2)_2$-; m.p. 245-248 °C, $M^+$ 475.1.

Example 13: X = 6-$CH_3O$; $X^1$ = 7-$CH_3O$; $X^2$ = H; $R_1$ = $CH_3$; $R_2$ = $C_6H_5CH_2$-; $R_3$ = H; and $R_4$ = 2,3-$(CH_3O)_2C_6H_3(CH_2)_2$-; m.p. 210-211 °C, $M^+$ 488.3.

Example 14: X = 6-$CH_3O$; $X^1$ = 7-$CH_3O$; $X^2$ = H; $R_1$ = $CH_3$; $R_2$ = $C_6H_5CH_2$-; $R_3$ = H; and $R_4$ = 2-$ClC_6H_4(CH_2)_2$-; m.p. 218-219 °C, $M^+$ 462.2.

Example 15: X = 6-$CH_3O$; $X^1$ = 7-$CH_3O$; $X^2$ = H; $R_1$ = $CH_3$; $R_2$ = 3,4-$(CH_3O)_2C_6H_3(CH_2)_2$-; $R_3$ = H; and $R_4$ = 2,3-$(CH_3O)_2C_6H_3(CH_2)_2$-; m.p. 83-86 °C, $M^+$ 563.4.

Example 16: X = 6-$CH_3O$; $X^1$ = 7-$CH_3O$; $X^2$ = H; $R_1$ = $CH_3$; $R_2$ = $C_6H_5CH_2$-; $R_3$ = H; and $R_4$ = 3-$CH_3OC_6H_4(CH_2)_2$-; m.p. 194-195 °C, $M^+$ 459.3.

Example 17: X = 6-$CH_3O$; $X^1$ = 7-$CH_3O$; $X^2$ = H; $R_1$ = $CH_3$; $R_2$ = $C_6H_5CH_2$-; $R_3$ = H; and $R_4$ = 2-Br-3,4-$(CH_3O)_2$-$C_6H_2(CH_2)_2$-; m.p. 219-220 °C, $M^+$ 569.0.

Example 18: X + $X^1$ = 6,7-$O_2CH_2$; $X^2$ = H; $R_1$ = $CH_3$; $R_2$ = $C_6H_5CH_2$-; $R_3$ = H; and $R_4$ = 3,4-$(CH_3O)$-$_2C_6H_3(CH_2)_2$-; m.p. 205-207 °C (free base), $M^+$473.0.

Example 19: X = 6-$CH_3O$; $X^1$ = 7-$CH_3O$; $X^2$ = H; $R_1$ = $C_2H_5$; $R_2$ = $C_6H_5CH_2$-; $R_3$ = H; and $R_4$ = 3,4-$(CH_3O)_2$-$C_6H_3(CH_2)_2$-; m.p. 171.5-172.5 °C, $M^+$ 503.3.

Example 20: X = 6-$CH_3O$; $X^1$ = 7-$CH_3O$; $X^2$ = H; $R_1$ = $C_2H_5$; $R_2$ = $C_6H_5CH_2$-; $R_3$ = H; and $R_4$ = 4-$C_2H_5OC_6H_4$-$(CH_2)_2$-; m.p. 192-194.5 °C, $M^+$ 473.5.

Example 21: X = 6-$CH_3O$; $X^1$ = 7-$CH_3O$; $X^2$ = 8-$CH_3O$; $R_1$ = $CH_3$; $R_2$ = $C_6H_5CH_2$-; $R_3$ = H; and $R_4$ = 3,4-$(CH_3O)_2$-$C_6H_3(CH_2)_2$-; m.p. 199-201 °C, $M^+$ 519.2.

Example 22: X, $X^1$ and $X^2$ = H; $R_1$ = $CH_3$; $R_2$ = $C_6H_5CH_2$-; $R_3$ = H; and $R_4$ = 3,4-$(CH_3O)_2C_6H_3(CH_2)_2$-; m.p. 95-98 °C (free base), $M^+$ 429.1.

Example 23: X = 6-$CH_3O$; $X^1$ = 7-$CH_3O$; $X^2$ = H; $R_1$ = $C_2H_5$; $R_2$ = $C_6H_5CH_2$-; $R_3$ = H; and $R_4$ = 4-$ClC_6H_4$-$(CH_2)_2$-; m.p. 210-212 °C, $M^+$ 463.2.

Example 24: X = 6-$CH_3O$; $X^1$ = 7-$CH_3O$; $X^2$ = H; $R_1$ = $CH_3$; $R_2$ = 4-$FC_6H_4CH_2$-; $R_3$ = H; and $R_4$ = 3,4-$(CH_3O)_2C_6H_3$-$(CH_2)_2$-; m.p. 185-187 °C, $M^+$ 507.0.

Example 25: X = 6-$CH_3O$; $X^1$ = 7-$CH_3O$; $X^2$ = H; $R_1$ = H; $R_2$ = 3,4-$(CH_3O)_2C_6H_3CH_2$-; $R_3$ = H; and $R_4$ = $C_6H_5CH_2$-; m.p. 144-145 °C (free base), $M^+$ 497.0.

Example 26: X = 6-$CH_3O$; $X^1$ = 7-$CH_3O$; $X^2$ = H; $R_1$ = H; $R_2$ = $C_6H_5CH_2$-; and

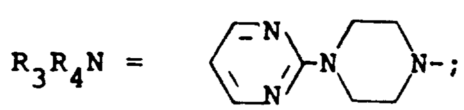

m.p. 278-281 °C, $M^+$ 458.0.

Example 27: X = 6-$CH_3O$; $X^1$ = 7-$CH_3O$; $X^2$ = H; $R_1$ = H; $R_2$ = $C_6H_5CH_2$-; and

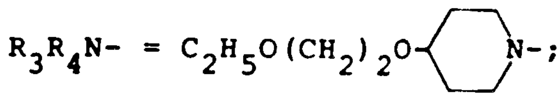

m.p. 213-215 °C, $M^+$ 467.0.

Example 28: X = 6-$CH_3O$; $X^1$ = 7-$CH_3O$; $X^2$ = H; $R_1$ = H; $R_2$ = $C_6H_5CH_2$-; $R_3$ = H; and $R_4$ = $C_6H_5CH_2$-;m.p. 196-199 °C, $M^+$ 542.0.

Example 29: X = 6-$CH_3O$; $X^1$ = 7-$CH_3O$; $X^2$ = H; $R_1$ = H; $R_2$ = 3,4-$(CH_3O)_2C_6H_3$-; and $R_3$, $R_4$ = $C_2H_5$; $M^+$ 493.4 (free base).

Example 30: X = 6-$C_2H_5O$; $X^1$ = 7-$C_2H_5O$; $X^2$ = H; $R_1$ = $CH_3$; $R_2$ = $C_6H_5CH_2$-; $R_3$ = H; and $R_4$ = 3,4-$(CH_3O)_2$-$C_6H_3(CH_2)_2$-; m.p. 187-188 °C, $M^+$ 517.5.

Example 31: X = 6-$CH_3O$; $X^1$ = 7-$CH_3O$; $X^2$ = H; $R_1$ = $CH_3$ $R_2$ = $C_6H_5CH_2$-; $R_3$ = $CH_3$; and $R_4$ = 3,4-$(CH_3O)_2C_6H_3$-$(CH_2)_2$-; m.p. 183-185 °C, $M^+$ 503.3.

Example 32: X and $X^1$ = H; $X^2$ = 8-$CH_3O$; $R_1$ = $CH_3$; $R_2$ = $C_6H_5CH_2$-; $R_3$ = H; and $R_4$ = 3,4-$(CH_3O)$-$_2C_6H_3(CH_2)_2$-, m.p. 162-164 °C (free base), $M^+$ 459.0.

Example 33: X and $X^1$ = H; $X^2$ = 8-$CH_3O$; $R_1$ = $CH_3$; $R_2$ = $C_6H_5CH_2$-; $R_3$ = H; and $R_4$ = 2-$ClC_6H_4$-$(CH_2)_2$-; m.p. 185-186 °C (free base), $M^+$ 433.0.

Example 34: X and $X^1$ = H; $X^2$ = 8-$CH_3O$; $R_1$ = $CH_3$; $R_2$ = $C_6H_5CH_2$-; $R_3$ = H; and $R_4$ = 2,3-$(CH_3O)$-$_2C_6H_3(CH_2)_2$-; m.p. 182-184.5 °C (free base), $M^+$ 459.0.

Example 35: X = 6-$CH_3O$; $X^1$ = 7-$CH_3O$; $X^2$ = H; $R_1$ = H; $R_2$ = $C_6H_5$-; and

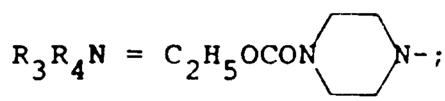

$M^+$ 438.0.

Example 36: X = 6-$CH_3O$; $X^1$ = 7-$CH_3O$; $X^2$ = H; $R_1$ = $CH_3$; $R_2$ = $CH_3(CH_2)_3$-; $R_3$ = H; and $R_4$ = 3,4-$(CH_3O)_2C_6H_3$-$(CH_2)_2$-; m.p. 160-162 °C, $M^+$ 455.2.

Example 37: X = 6-$CH_3O$; $X^1$ = 7-$CH_3O$; $X^2$ = H; $R_1$ = $CH_3$; $R_2$ = 2,3-$(CH_3O)_2C_6H_3CH_2$-; $R_3$ = H; and $R_4$ = 3,4-$(CH_3O)_2$-$C_6H_3(CH_2)_2$-; m.p. 103-105 °C, $M^+$ 549.2.

PREPARATION A

Starting with the requisite reagents and employing the procedure of Example 1A, the following intermediates were prepared:

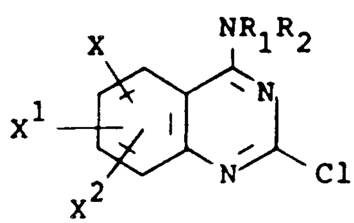

| X | $X^1$ | $X^2$ | $R_1R_2N$ | m.p., °C |
|---|---|---|---|---|
| 6-$CH_3O$ | 7-$CH_3O$ | H | 2-$CH_3OC_6H_4CH_2NCH_3$ | 168-171.5 |
| 6-$CH_3O$ | 7-$CH_3O$ | H | 3,4-$(CH_3O)_2C_6H_3NH$- | 250-251 |
| H | H | H | $C_6H_5NH$- | 189-191 |
| 6-$CH_3O$ | 7-$CH_3O$ | H | 3,4-$(CH_3O)_2C_6H_3NCH_3$ | 216-218 |
| 6-$CH_3O$ | 7-$CH_3O$ | H | $C_6H_5NH$- | 220-222 |
| 6-$CH_3O$ | 7-$CH_3O$ | H | 3-Cl-4-$FC_6H_3CH_2NCH_3$ | 152-154 |
| 6-$CH_3O$ | 7-$CH_3O$ | H | 2,6-$(CH_3O)_2C_6H_3CH_2NCH_3$ | 155-158 |
| 6-$CH_3O$ | 7-$CH_3O$ | H | $C_6H_5CH_2NH$- | 209-210 |
| 6-$CH_3O$ | 7-$CH_3O$ | H | $C_6H_5CH_2NCH_3$- | 187-188 |
| 6-$CH_3O$ | 7-$CH_3O$ | H | 3,4-$(CH_3O)_2C_6H_3(CH_2)_2NCH_3$ | 132-136 |

| X | $X^1$ | $X^2$ | $R_1R_2N$ | m.p., °C |
|---|---|---|---|---|
| 6,7-$OCH_2O$- | | H | $C_6H_5CH_2NCH_3$ | 155-157 |
| 6-$CH_3O$ | 7-$CH_3O$ | H | $C_6H_5CH_2NC_2H_5$ | 128-130 |
| 6-$CH_3O$ | 7-$CH_3O$ | 8-$CH_3O$ | $C_6H_5CH_2NCH_3$ | 122-123 |
| H | H | H | $C_6H_5CH_2NCH_3$ | 98-99 |
| 6-$CH_3O$ | 7-$CH_3O$ | H | 4-$FC_6H_4CH_2NCH_3$ | 175-177 |
| 6-$CH_3O$ | 7-$CH_3O$ | H | 3,4-$(CH_3O)_2C_6H_3CH_2NH$- | |
| 6-$C_2H_5O$ | 7-$C_2H_5O$ | H | $C_6H_5CH_2NCH_3$ | 159-160 |
| H | H | 8-$CH_3O$ | $C_6H_5CH_2NCH_3$ | 115-115.5 |
| 6-$CH_3O$ | 7-$CH_3O$ | H | $CH_3(CH_2)_3NCH_3$ | 180-182 |
| 6-$CH_3O$ | 7-$CH_3O$ | H | 2,3-$(CH_3O)_2C_6H_3CH_2NCH_3$ | |

## Claims

### Claims for the following Contracting States : AT, BE, CH, DE, DK, FR, GB, IT, LI, LU, NL, SE

**1.** A compound of the formula

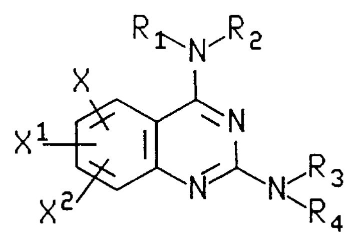

or a pharmaceutically acceptable acid addition salt thereof, wherein X is alkyl having one to three carbon atoms, alkoxy having one to three carbon atoms, chloro, fluoro, amino, alkylamino having one to three carbon atoms, dialkylamino having two to six carbon atoms or trifluoromethyl; $X^1$ is hydrogen, alkyl having one to three carbon atoms, alkoxy of one to three carbon atoms, fluoro, chloro or dialkylamino having two to six carbon atoms; $X_2$ is hydrogen, alkyl having one to three carbon atoms or alkoxy having one to three carbon atoms; X and $X^1$ when taken together are ethylenedioxy or methylenedioxy; $R_1$ is hydrogen or alkyl having one to three carbon atoms; $R_2$ is alkyl having one to ten carbon atoms or a moiety of the formula

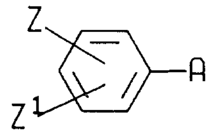

where Z and $Z^1$ are each hydrogen, alkyl having one to three carbon atoms, alkoxy having one to three carbon atoms, fluoro, chloro, bromo, trifluoromethyl or dialkylamino having two to six carbon atoms, A is a chemical bond or alkylene having from one to four carbon atoms and Z and $Z^1$ when taken together are ethylenedioxy or methylenedioxy; $R_3$ is hydrogen or alkyl having one to three carbon atoms; $R_4$ is aralkyl of the formula

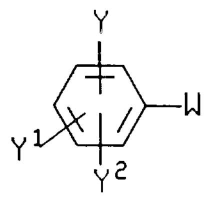

where W is alkylene having one to four carbon atoms, Y and $Y^1$ are each hydrogen, alkyl having one to three carbon atoms, alkoxy having one to three carbon atoms, fluoro, chloro, bromo, trifluoromethyl or dialkylamino having two to six carbon atoms, $Y^2$ is hydrogen, alkyl having one to three carbon atoms or alkoxy having one to three carbon atoms and $Y^1$ and $Y^2$ when taken together are ethylenedioxy or methylenedioxy;

and $R_3$ and $R_4$ when taken together with the nitrogen to which they are attached are 4-phenylpiperazino, 4-alkoxyethoxypiperidino said alkoxy having from one to three carbon atoms or 4-alkoxycarbonylpiperizino said alkoxy having one to three carbon atoms, with the proviso that:

when $R_1$ is alkyl having one to three carbons then $R_2$ is not alkyl having one to eight carbons;

when $X^1$ and $X^2$ are each hydrogen X is amino, alkylamino having one to three carbon atoms, dialkylamino having two to six carbon atoms or trifluoromethyl;

when $X^1$ is H and $X^2$ is methoxy or $X^1$ is methoxy and $X^2$ is hydrogen, X is methoxy, $R_1$ is ethyl, $R_3$ is hydrogen, $R_4$ is the aralkyl where W is ethylene and Y, $Y^1$ and $Y^2$ are each hydrogen, then $R_2$ is not ethyl; and

when $R_1$ is alkyl having one to three carbons and $R_2$ is a moiety of the formula

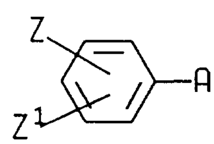

where A is $-CH_2-$, then Z and $Z^1$ cannot both be hydrogen.

**2.** A compound of claim 1, wherein X and $X^1$ are each methoxy, $R_2$ is aralkyl of the formula

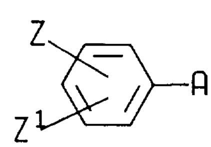

where Z is hydrogen, $R_3$ is hydrogen and $R_4$ is aralkyl of the formula

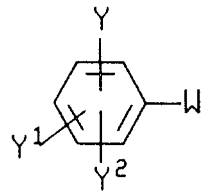

where W is $-(CH_2)_2-$, Y and $Y^1$ are each methoxy and $Y^2$ is hydrogen.

3. The compound of claim 2, wherein $R_1$ is methyl, X is 6-methoxy, $X^1$ is 7-methoxy, $X^2$ is hydrogen, $Z^1$ is 2-methoxy, A is $-CH_2-$, Y is 3-methoxy and $Y^1$ is 4-methoxy.

4. The compound of claim 2, wherein $R_1$ is hydrogen, X is 6-methoxy, $X^1$ is 7-methoxy, $X^2$ is hydrogen, $Z^1$ is hydrogen, A is $-CH_2-$, Y is 3-methoxy and $Y^1$ is 4-methoxy.

5. The compound of claim 2, wherein $R_1$ is methyl, X is 6-methoxy, $X^1$ is 7-methoxy, $X^2$ is hydrogen, $Z^1$ is hydrogen, A is a chemical bond, Y is 3-methoxy and $Y^1$ is 4-methoxy.

6. The compound of claim 2, wherein $R_1$ is methyl, X is 6-methoxy, $X^1$ is 7-methoxy, $X^2$ is hydrogen, $Z^1$ is 4-fluoro, A is $-CH_2-$, Y is 3-methoxy and $Y^1$ is 4-methoxy.

7. A compound of claim 1, wherein $X^2$ is hydrogen, $R_1$ is methyl, $R_2$ is aralkyl of the formula

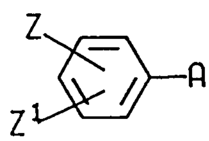

$R_3$ is hydrogen and $R_4$ is aralkyl of the formula

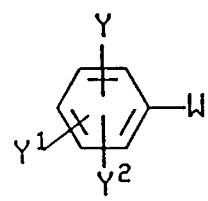

where W is $-(CH_2)_2-$, Y and $Y^1$ are each methoxy and $Y^2$ is hydrogen.

8. The compound of claim 7, wherein X is 6-methoxy, $X^1$ is 7-methoxy, Z is 3-methoxy, $Z^1$ is 4-methoxy, A is a chemical bond, Y is 2-methoxy and $Y^1$ is 3-methoxy.

9. The compound of claim 7, wherein X is 6-methoxy, $X^1$ is 7-methoxy, Z is 3-methoxy, $Z^1$ is 4-methoxy, A is a chemical bond, Y is 3-methoxy and $Y^1$ is 4-methoxy.

10. The compound of claim 7, wherein X is 6-methoxy, $X^1$ is 7-methoxy, Z is 2-methoxy, $Z^1$ is 3-methoxy, A is $-CH_2-$, Y is 3-methoxy and $Y^1$ is 4-methoxy.

11. The use of a compound according to Claim 1, for the manufacture of a medicament useful for treatment or prophylaxis of diseases or disorders affecting mammals mediated by a P-glycoprotein.

12. The use according to Claim 11, wherein the mammal is a human suffering from cancer, and said medicament is administered before, with or after a chemotherapeutic agent.

**13.** A compound according to any one of claims 1 to 10 or a pharmaceutically acceptable acid addition salt thereof for use as a medicament.

**14.** A pharmaceutical composition for administration to a mammal which comprises a P-glycoprotein inhibiting amount of a compound of claim 1, a pharmaceutically acceptable carrier and optionally, an anticancer effective amount of a chemotherapeutic agent.

**15.** A process for preparing a compound of the formula

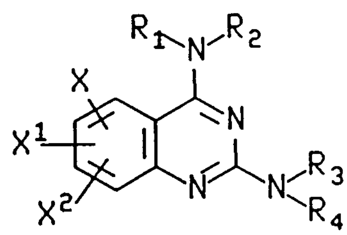

and a pharmaceutically acceptable acid addition salt thereof, wherein X is alkyl having one to three carbon atoms, alkoxy having one to three carbon atoms, chloro, fluoro, amino, alkylamino having one to three carbon atoms, dialkylamino having two to six carbon atoms or trifluoromethyl; $X^1$ is hydrogen, alkyl having one to three carbon atoms, fluoro, chloro or dialkylamino having two to six carbon atoms; $X^2$ is hydrogen, alkyl having one to three carbon atoms or alkoxy having one to three carbon atoms; X and $X^1$ when taken together are ethylenedioxy or methylenedioxy; $R_1$ is hydrogen or alkyl having one to three carbon atoms; $R_2$ is alkyl having one to ten carbon atoms or a moiety of the formula

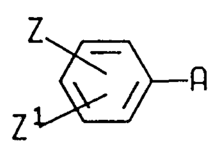

where Z and $Z^1$ are each hydrogen, alkyl having one to three carbon atoms, alkoxy having one to three carbon atoms, fluoro, chloro, bromo, trifluoromethyl or dialkylamino having two to six carbon atoms, A is a chemical bond or alkylene having from one to four carbon atoms and Z and $Z^1$ when taken together are ethylenedioxy or methylenedioxy; $R_3$ is hydrogen or alkyl having one to three carbon atoms; $R_4$ is aralkyl of the formula

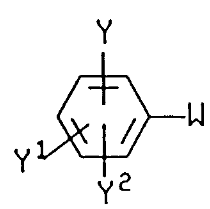

where W is alkylene having one to four carbon atoms, Y and $Y^1$ are each hydrogen, alkyl having one to three carbon atoms, alkoxy having one to three carbon atoms, fluoro, chloro, bromo, trifluoromethyl or dialkylamino having two to six carbon atoms, $Y^2$ is hydrogen, alkyl having one to three carbon atoms or alkoxy having one to three carbon atoms and $Y^1$ and $Y^2$ when taken together are ethylenedioxy or methylenedioxy; and $R_3$ and $R_4$ when taken together with the nitrogen to which they are attached are 4-phenylpiperazino, 4-alkoxyethoxypiperidino said alkoxy having from one to three carbon atoms or 4-alkoxycarbonylpiperizino said alkoxy having from one to three carbon atoms, with the proviso that:
when $R_1$ is alkyl having one to three carbons then $R_2$ is not alkyl having one to eight carbons;
when $X^1$ and $X^2$ are each hydrogen X is amino, alkylamino having one to three carbon atoms, dialkylamino having two to six carbon atoms or trifluoromethyl; and when $X^1$ is H and $X^2$ is methoxy or

$X^1$ is methoxy and $X^2$ is hydrogen, X is methoxy, $R_1$ is ethyl, $R_3$ is hydrogen, $R_4$ is the aralkyl where W is ethylene and Y, $Y^1$ and $Y^2$ are each hydrogen, then $R_2$ is not ethyl; and when $R_1$ is alkyl having one to three carbons and $R_2$ is a moiety of the formula

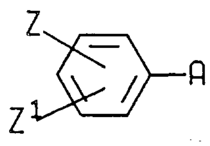

where A is $-CH_2-$, then Z and $Z^1$ cannot both be hydrogen, which comprises reacting a compound of the formula

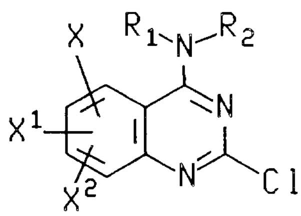

wherein $R_1$, $R_2$, X, $X^1$ and $X^2$ are defined with a compound of the formula

$R_3R_4NH$

where $R_3$ and $R_4$ are defined in a reaction-inert solvent containing one equivalent of an amine-acid scavenger at a reaction temperature of 100-200 °C until the reaction is substantially complete.

**Claims for the following Contracting States : ES, GR**

1. A process for preparing a compound of the formula

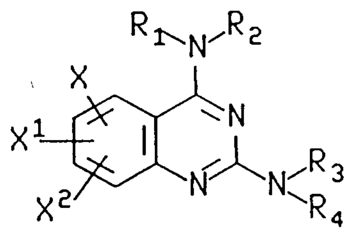

and a pharmaceutically acceptable acid addition salt thereof, wherein X is alkyl having one to three carbon atoms, alkoxy having one to three atoms, chloro, fluoro, amino, alkylamino having one to three carbon atoms, dialkylamino having two to six carbon atoms or trifluoromethyl; $X^1$ is hydrogen, alkyl having one to three carbon atoms, fluoro, chloro, or dialkylamino having two to six carbon atoms; $X^2$ is hydrogen, alkyl having one to three carbon atoms or alkoxy having one to three carbon atoms; X and $X^1$ when taken together are ethylenedioxy or methylenedioxy; is hydrogen or alkyl having one to three carbon atoms; $R_2$ is alkyl having one to ten carbon atoms or a moiety of the formula

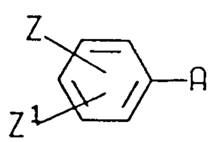

where Z and $Z^1$ are each hydrogen, alkyl having one to three carbon atoms, alkoxy having one to three carbon atoms, fluoro, chloro, bromo, trifluoromethyl or dialkylamino having two to six carbon atoms, A is a chemical bond or alkylene having from one to four carbon atoms and Z and $Z^1$ when taken together are ethylenedioxy or methylenedioxy; $R_3$ is hydrogen or alkyl having one to three carbon atoms; $R_4$ is aralkyl or the formula

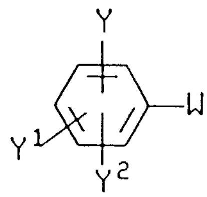

where W is alkylene having one to four carbon atoms, Y and $Y^1$ are each hydrogen alkyl having one to three carbon atoms, alkoxy having one to three carbon atoms, fluoro, chloro, bromo, trifluoromethyl or dialkylamino having two to six carbon atoms, $Y^2$ is hydrogen alkyl having one to three carbon atoms or alkoxy having one to three carbon atoms and $Y^1$ and $Y^2$ when taken together are ethylenedioxy or methylenedioxy; and $R_3$ and $R_4$ when taken together with the nitrogen to which they are attached are 4-phenylpiperazino, 4-alkoxyethoxpiperidino said alkoxy having from one to three carbon atoms or 4-alkoxycarbonylpiperizino said alkoxy having from one to three carbon atoms, with the proviso that:
when $R_1$ is alkyl having one to three carbons then $R_2$ is not alkyl having one to eight carbons;
when $X^1$ and $X^2$ are each hydrogen X is amino, alkylamino having one to three carbon atoms, dialkylamino having two to six carbon atoms or trifluoromethyl; and when $X^1$ is H and $X^2$ is methoxy or $X^1$ is methoxy and $X^2$ is hydrogen, X is methoxy, $R_1$ is ethyl, $R_3$ is hydrogen, $R_4$ is the aralkyl where W is ethylene and Y, $Y^1$ and $Y^2$ are each hydrogen, then $R_2$ is not ethyl; and when $R_1$ is alkyl having one to three carbons and $R_2$ is a moiety of the formula

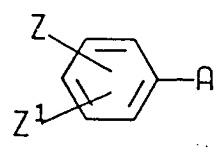

where A is $-CH_2-$, then Z and $Z^1$ cannot both be hydrogen, which comprises reacting a compound of the formula

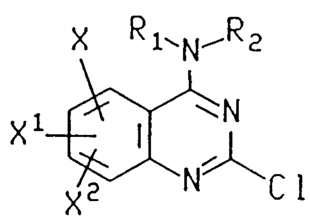

wherein $R_1$, $R_2$, X, $X^1$ and $X^2$ are defined with a compound of the formula

$R_3R_4NH$

where $R_3$ and $R_4$ are defined in a reaction-inert solvent containing one equivalent of a tertiary amine-acid scavenger at a reaction temperature of 100-200 °C until the reaction is substantially complete.

**2.** A process of claim 1, wherein X and $X^1$ are each methoxy, $R_2$ is aralkyl of the formula

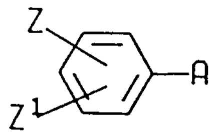

where Z is hydrogen, $R_3$ is hydrogen and $R_4$ is aralkyl of the formula

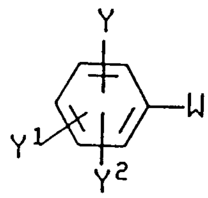

where W is $-(CH_2)_2-$, Y and $Y^1$ are each methoxy and $Y^2$ is hydrogen.

**3.** A process of claim 2, wherein $R_1$ is methyl, X is 6-methoxy, $X^1$ is 7-methoxy, $X^2$ is hydrogen, $Z^1$ is 2-methoxy, A is $-CH_2-$, Y is 3-methoxy and $Y^1$ is 4-methoxy.

**4.** A process of claim 2, wherein $R_1$ is hydrogen, X is 6-methoxy, $X^1$ is 7-methoxy, $X^2$ is hydrogen, $Z^1$ is hydrogen, A is $-CH_2-$, Y is 3-methoxy and $Y^1$ is 4-methoxy.

**5.** A process of claim 2, wherein $R_1$ is methyl, X is 6-methoxy, $X^1$ is 7-methoxy, $X^2$ is hydrogen, $Z^1$ is hydrogen, A is a chemical bond, Y is 3-methoxy and $Y^1$ is 4-methoxy.

**6.** A process of claim 2, wherein $R_1$ is methyl, X is 6-methoxy, $X^1$ is 7-methoxy, $X^2$ is hydrogen, $Z^1$ is 4-fluoro, A is $-CH_2-$, Y is 3-methoxy and $Y^1$ is 4-methoxy.

**7.** A process of claim 1, wherein $X^2$ is hydrogen, $R_1$ is aralkyl of the formula

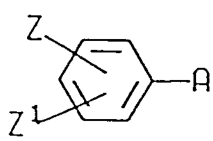

$R_3$ is hydrogen and $R_4$ is aralkyl of the formula

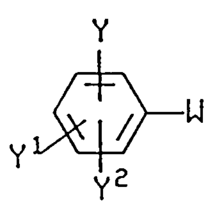

where W is $-(CH_2)_2-$, Y and $Y^1$ are each methoxy and $Y^2$ is hydrogen.

**8.** A process of claim 7, wherein X is 6-methoxy, $X^1$ is 7-methoxy, Z is 3-methoxy, $Z^1$ is 4-methoxy, A is a chemical bond, Y is 2-methoxy and $Y^1$ is 3-methoxy.

9. A process of claim 7, wherein X is 6-methoxy, $X^1$ is 7-methoxy, Z is 3-methoxy, $Z^1$ is 4-methoxy, A is a chemical bond, Y is 3-methoxy and $Y^1$ is 4-methoxy.

10. A process of claim 7, wherein X is 6-methoxy, $X^1$ is 7-methoxy, Z is 2-methoxy, $Z^1$ is 3-methoxy, A is $-CH_2-$, Y is 3-methoxy and $Y^1$ is 4-methoxy.

**Patentansprüche**
**Patentansprüche für folgende Vertragsstaaten : AT, BE, CH, DE, DK, FR, GB, IT, LI, LU, NL, SE**

1. Verbindung der Formel

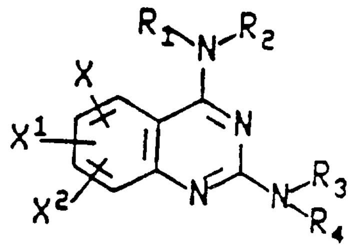

oder ein pharmazeutisch annehmbares Säure-Additionssalz davon, worin X ein Alkylrest mit 1 bis 3 Kohlenstoffatomen, ein Alkoxyrest mit 1 bis 3 Kohlenstoffatomen, Chlor, Fluor, ein Aminorest, ein Alkylaminorest mit 1 bis 3 Kohlenstoffatomen, ein Dialkylaminorest mit 2 bis 6 Kohlenstoffatomen oder ein Trifluormethylrest ist; $X^1$ Wasserstoff, ein Alkylrest mit 1 bis 3 Kohlenstoffatomen, Fluor, Chlor oder ein Dialkylaminorest mit 2 bis 6 Kohlenstoffatomen ist; $X^2$ Wasserstoff, ein Alkylrest mit 1 bis 3 Kohlenstoffatomen oder ein Alkoxyrest mit 1 bis 3 Kohlenstoffatomen ist; X und $X^1$, wenn sie zusammengenommen werden, einen Ethylendioxy- oder Methylendioxyrest bilden; $R_1$ Wasserstoff oder ein Alkylrest mit 1 bis 3 Kohlenstoffatomen ist; $R_2$ ein Alkylrest mit 1 bis 10 Kohlenstoffatomen oder ein Aralkylrest der Formel

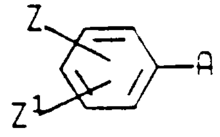

ist, worin Z und $Z^1$ jeweils Wasserstoff, Alkylreste mit 1 bis 3 Kohlenstoffatomen, Alkoxyreste mit 1 bis 3 Kohlenstoffatomen, Fluor, Chlor, Brom, Trifluormethylreste oder Dialkylaminoreste mit 2 bis 6 Kohlenstoffatomen sind, A eine chemische Bindung oder ein Alkylenrest mit 1 bis 4 Kohlenstoffatomen ist und Z und $Z^1$, wenn sie zusammengenommen werden, einen Ethylendioxy- oder Methylendioxyrest bilden; $R_3$ Wasserstoff oder ein Alkylrest mit 1 bis 3 Kohlenstoffatomen ist; $R_4$ ein Aralkylrest der Formel:

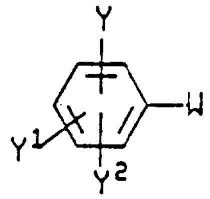

ist, worin W ein Alkylenrest mit 1 bis 4 Kohlenstoffatomen ist, Y und $Y^1$ jeweils Wasserstoff, Alkylreste mit 1 bis 3 Kohlenstoffatomen, Alkoxyreste mit 1 bis 3 Kohlenstoffatomen, Fluor, Chlor, Brom, Trifluormethylreste oder Dialkylaminoreste mit 2 bis 6 Kohlenstoffatomen sind, $Y^2$ Wasserstoff, ein Alkylrest mit 1 bis 3 Kohlenstoffatomen oder ein Alkoxyrest mit 1 bis 3 Kohlenstoffatomen ist und $Y^1$

und $Y^2$, wenn sie zusammengenommen werden, einen Ethylendioxy- oder Methylendioxyrest bilden; und $R_3$ und $R_4$, wenn sie zusammengenommen werden mit dem Stickstoff, an den sie gebunden sind, einen 4-Phenylpiperazinorest, 4-Alkoxyethoxypiperidinorest, dessen Alkoxyrest 1 bis 3 Kohlenstoffatome hat, oder einen 4-Alkoxycarbonylpiperizinorest, dessen Alkoxyrest 1 bis 3 Kohlenstoffatome hat, bilden, mit dem Vorbehalt, daß dann wenn $R_1$ ein Alkylrest mit 1 bis 3 Kohlenstoffatomen ist, $R_2$ kein Alkylrest mit 1 bis 8 Kohlenstoffatomen ist,
wenn $X^1$ und $X^2$ jeweils Wasserstoff sind, X ein Aminorest, ein Alkylaminorest mit 1 bis 3 Kohlenstoffatomen, ein Dialkylaminorest mit 2 bis 6 Kohlenstoffatomen oder ein Trifluormethylrest ist,
wenn $X^1$ H ist und $X^2$ ein Methoxyrest ist oder $X^1$ ein Methoxyrest und $X^2$ Wasserstoff ist, X ein Methoxyrest ist, $R_1$ ein Ethylrest ist, $R_3$ Wasserstoff ist, $R_4$ der Aralkylrest ist, worin W ein Ethylenrest ist und Y, $Y^1$ und $Y^2$ jeweils Wasserstoff sind, dann $R_2$ nicht Ethyl ist, und
wenn $R_1$ ein Alkylrest mit 1 bis 3 Kohlenstoffatomen ist und $R_2$ ein Teil der Formel

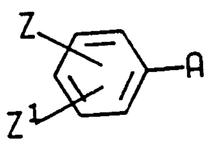

ist, worin A $-CH_2-$ ist, dann Z und Z' nicht beide Wasserstoff sein können.

**2.** Verbindung nach Anspruch 1, worin X und $X^1$ jeweils Methoxyreste sind, $R_2$ ein Aralkylrest der Formel

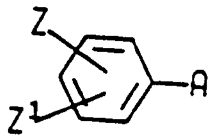

ist, worin Z Wasserstoff ist, $R_3$ Wasserstoff ist und $R_4$ ein Aralkylrest der Formel:

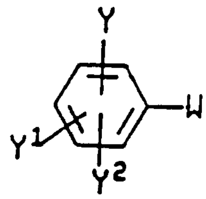

ist, worin W $-(CH_2)_2-$ ist, Y und $Y^1$ jeweils Methoxyreste sind und $Y^2$ Wasserstoff ist.

**3.** Verbindung nach Anspruch 2, worin $R_1$ ein Methylrest ist, X ein 6-Methoxyrest ist, $X^1$ ein 7-Methoxyrest ist, $X^2$ Wasserstoff ist, $Z^1$ ein 2-Methoxyrest ist, A $-CH_2-$ ist, Y ein 3-Methoxyrest ist und $Y^1$ ein 4-Methoxyrest ist.

**4.** Verbindung nach Anspruch 2, worin $R_1$ Wasserstoff ist, X ein 6-Methoxyrest ist, $X^1$ ein 7-Methoxyrest ist, $X^2$ Wasserstoff ist, $Z^1$ Wasserstoff ist, A $-CH_2-$ ist, Y ein 3-Methoxyrest ist und $Y^1$ 4-Methoxyrest ist.

**5.** Verbindung nach Anspruch 2, worin $R_1$ ein Methylrest ist, X ein 6-Methoxyrest ist, $X^1$ ein 7-Methoxyrest ist, $X^2$ Wasserstoff ist, $Z^1$ Wasserstoff ist, A eine chemische Bindung ist, Y ein 3-Methoxyrest ist und $Y^1$ ein 4-Methoxyrest ist.

**6.** Verbindung nach Anspruch 2, worin $R_1$ ein Methylrest ist, X ein 6-Methoxyrest ist, $X^1$ ein 7-Methoxyrest ist, $X^2$ Wasserstoff ist, $Z^1$ ein 4-Fluorrest ist, A $-CH_2-$ ist, Y ein 3-Methoxyrest ist und $Y^1$ ein 4-Methoxyrest ist.

**7.** Verbindung nach Anspruch 1, worin $X^2$ Wasserstoff ist, $R_1$ ein Methylrest ist, $R_2$ ein Aralkylrest der Formel

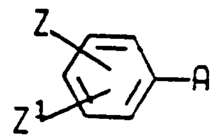

ist, $R_3$ Wasserstoff ist und $R_4$ ein Aralkylrest der Formel

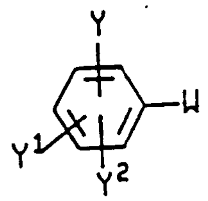

ist, worin W $-(CH_2)_2-$ ist, Y und $Y^1$ jeweils ein Methoxyrest sind und $Y^2$ Wasserstoff ist.

**8.** Verbindung nach Anspruch 7, worin X ein 6-Methoxyrest ist, $X^1$ ein 7-Methoxyrest ist, Z ein 3-Methoxyrest ist, $Z^1$ ein 4-Methoxyrest ist, A eine chemische Bindung ist, Y ein 2-Methoxyrest ist und $Y^1$ 3-Methoxyrest ist.

**9.** Verbindung nach Anspruch 7, worin X ein 6-Methoxyrest ist, $X^1$ ein 7-Methoxyrest ist, Z ein 3-Methoxyrest, $Z^1$ ein 4-Methoxyrest ist, A eine chemische Bindung ist, Y ein 3-Methoxyrest ist und $Y^1$ ein 4-Methoxyrest ist.

**10.** Verbindung nach Anspruch 7, worin X ein 6-Methoxyrest ist, $X^1$ ein 7-Methoxyrest ist, Z ein 2-Methoxyrest ist, Z' ein 3-Methoxyrest ist, A $-CH_2-$ ist, Y ein 3-Methoxyrest ist und $Y^1$ ein 4-Methoxyrest ist.

**11.** Verwendung einer Verbindung nach Anspruch 1 bei der Herstellung eines Arzneimittels, das zur Behandlung oder Prophylaxe von Erkrankungen oder Störungen bei einem Säugetier, die durch ein P-Glycoprotein vermittelt werden, geeignet ist.

**12.** Verwendung nach Anspruch 11, bei der das Säugetier ein an Krebs leidender Mensch ist und das Arzneimittel vor, mit oder nach einem chemotherapeutischen Mittel verabreicht wird.

**13.** Verbindung nach einem der Ansprüche 1 bis 10 oder ein pharmazeutisch annehmbares Säureadditionssalz davon zur Verwendung als Arzneimittel.

**14.** Pharmazeutische Zusammensetzung zur Verabreichung an ein Säugetier, die eine P-Glycoprotein inhibierende Menge einer Verbindung nach Anspruch 1, einen pharmazeutisch annehmbaren Träger und fakultativ eine gegen Krebs wirksame Menge eines chemotherapeutischen Mittels umfaßt.

**15.** Verfahren zur Herstellung einer Verbindung der Formel

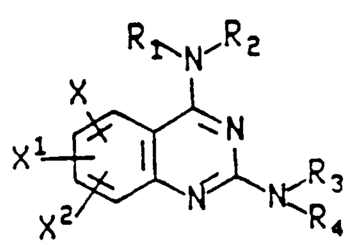

und eines pharmazeutisch annehmbaren Säureadditionssalzes davon, worin X ein Alkylrest mit 1 bis 3 Kohlenstoffatomen, ein Alkoxyrest mit 1 bis 3 Kohlenstoffatomen, Chlor, Fluor, ein Aminorest, ein Alkylaminorest mit 1 bis 3 Kohlenstoffatomen, ein Dialkylaminorest mit 2 bis 6 Kohlenstoffatomen oder ein Trifluormethylrest ist; $X^1$ Wasserstoff, ein Alkylrest mit 1 bis 3 Kohlenstoffatomen, Fluor, Chlor oder ein Dialkylaminorest mit 2 bis 6 Kohlenstoffatomen ist; $X^2$ Wasserstoff, ein Alkylrest mit 1 bis 3 Kohlenstoffatomen oder ein Alkoxyrest mit 1 bis 3 Kohlenstoffatomen ist; X und $X^1$, wenn sie zusammengenommen werden, einen Ethylendioxy- oder Methylendioxyrest bilden; $R_1$ Wasserstoff oder ein Alkylrest mit 1 bis 3 Kohlenstoffatomen ist; $R_2$ ein Alkylrest mit 1 bis 10 Kohlenstoffatomen oder ein Aralkylrest der Formel

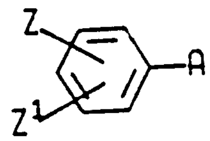

ist, worin Z und $Z^1$ jeweils Wasserstoff, Alkylreste. mit 1 bis 3 Kohlenstoffatomen, Alkoxyreste mit 1 bis 3 Kohlenstoffatomen, Fluor, Chlor, Brom, Trifluormethylreste oder Dialkylaminoreste mit 2 bis 6 Kohlenstoffatomen sind, A eine chemische Bindung oder ein Alkylenrest mit 1 bis 4 Kohlenstoffatomen ist und Z und $Z^1$, wenn sie zusammengenommen werden, einen Ethylendioxy- oder Methylendioxyrest bilden; $R_3$ Wasserstoff oder ein Alkylrest mit 1 bis 3 Kohlenstoffatomen ist; $R_4$ ein Aralkylrest der Formel:

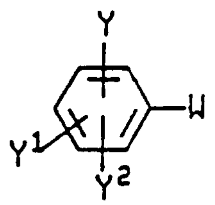

ist, worin W ein Alkylenrest mit 1 bis 4 Kohlenstoffatomen ist, Y und $Y^1$ jeweils Wasserstoff, Alkylreste mit 1 bis 3 Kohlenstoffatomen, Alkoxyreste mit 1 bis 3 Kohlenstoffatomen, Fluor, Chlor, Brom, Trifluormethylreste oder Dialkylaminoreste mit 2 bis 6 Kohlenstoffatomen sind, $Y^2$ Wasserstoff, ein Alkylrest mit 1 bis 3 Kohlenstoffatomen oder ein Alkoxyrest mit 1 bis 3 Kohlenstoffatomen ist und $Y^1$ und $Y^2$, wenn sie zusammengenommen werden, einen Ethylendioxy- oder Methylendioxyrest bilden; und $R_3$ und $R_4$, wenn sie zusammengenommen werden mit dem Stickstoff, an den sie gebunden sind, einen 4-Phenylpiperazinorest, 4-Alkoxyethoxypiperidinorest, dessen Alkoxyrest 1 bis 3 Kohlenstoffatome hat, oder einen 4-Alkoxycarbonylpiperizinorest, dessen Alkoxyrest 1 bis 3 Kohlenstoffatome hat, bilden, mit dem Vorbehalt, daß dann, wenn $R_1$ ein Alkylrest mit 1 bis 3 Kohlenstoffatomen ist, $R_2$ kein Alkylrest mit 1 bis 8 Kohlenstoffatomen ist,
wenn $X^1$ und $X^2$ jeweils Wasserstoff sind, X ein Aminorest, ein Alkylaminorest mit 1 bis 3 Kohlenstoffatomen, ein Dialkylaminorest mit 2 bis 6 Kohlenstoffatomen oder ein Trifluormethylrest ist,
und wenn $X^1$ H ist und $X^2$ ein Methoxyrest ist oder $X^1$ ein Methoxyrest und $X^2$ Wasserstoff ist, X ein Methoxyrest ist, $R_1$ ein Ethylrest ist, $R_3$ Wasserstoff ist, $R_4$ der Aralkylrest ist, worin W ein Ethylenrest ist und Y, $Y^1$ und $Y^2$ jeweils Wasserstoff sind, dann $R_2$ nicht Ethyl ist, und
wenn $R_1$ ein Alkylrest mit 1 bis 3 Kohlenstoffatomen ist und $R_2$ ein Teil der Formel

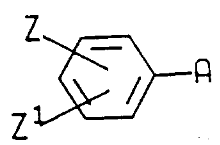

ist, worin A $-CH_2-$ ist, dann Z und Z' nicht beide Wasserstoff sein können,
das umfaßt das Umsetzen einer Verbindung der Formel

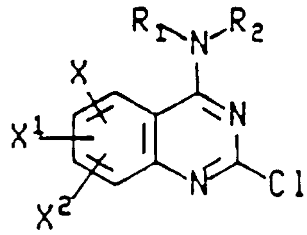 ,

worin $R_1$, $R_2$, X, $X^1$ und $X^2$ wie oben definiert sind, mit einer Verbindung der Formel

$R_3R_4NH$,

worin $R_3$ und $R_4$ wie oben definiert sind, in einem reaktionsinerten Lösungsmittel, das ein Äquivalent eines Amins als Säurefänger umfaßt, bei einer Reaktionstemperatur von 100 bis 200 °C, bis die Reaktion im wesentlichen vollständig ist.

**Patentansprüche für folgende Vertragsstaaten : ES, GR**

**1.** Verfahren zur Herstellung einer Verbindung der Formel

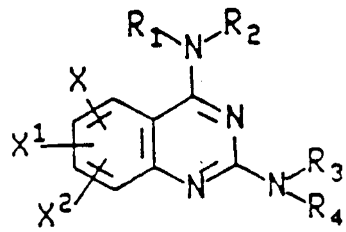

und eines pharmazeutisch annehmbaren SäureadditionsSalzes davon, worin X ein Alkylrest mit 1 bis 3 Kohlenstoffatomen, ein Alkoxyrest mit 1 bis 3 Kohlenstoffatomen, Chlor, Fluor, ein Aminorest, ein Alkylaminorest mit 1 bis 3 Kohlenstoffatomen, ein Dialkylaminorest mit 2 bis 6 Kohlenstoffatomen oder ein Trifluormethylrest ist; $X^1$ Wasserstoff, ein Alkylrest mit 1 bis 3 Kohlenstoffatomen, Fluor, Chlor oder ein Dialkylaminorest mit 2 bis 6 Kohlenstoffatomen ist; $X^2$ Wasserstoff, ein Alkylrest mit 1 bis 3 Kohlenstoffatomen oder ein Alkoxyrest mit 1 bis 3 Kohlenstoffatomen ist; X und $X^1$, wenn sie zusammengenommen werden, einen Ethylendioxy- oder Methylendioxyrest bilden; $R_1$ Wasserstoff oder ein Alkylrest mit 1 bis 3 Kohlenstoffatomen ist; $R_2$ ein Alkylrest mit 1 bis 10 Kohlenstoffatomen oder ein Aralkylrest der Formel

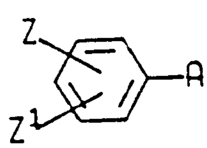

ist, worin Z und $Z^1$ jeweils Wasserstoff, Alkylreste mit 1 bis 3 Kohlenstoffatomen, Alkoxyreste mit 1 bis 3 Kohlenstoffatomen, Fluor, Chlor, Brom, Trifluormethylreste oder Dialkylaminoreste mit 2 bis 6 Kohlenstoffatomen sind, A eine chemische Bindung oder ein Alkylenrest mit 1 bis 4 Kohlenstoffatomen ist und Z und $Z^1$, wenn sie zusammengenommen werden, einen Ethylendioxy- oder Methylendioxyrest bilden; $R_3$ Wasserstoff oder ein Alkylrest mit 1 bis 3 Kohlenstoffatomen ist; $R_4$ ein Aralkylrest der Formel:

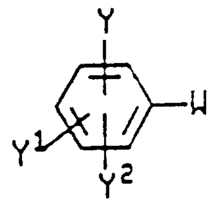

ist, worin W ein Alkylenrest mit 1 bis 4 Kohlenstoffatomen ist, Y und $Y^1$ jeweils Wasserstoff, Alkylreste mit 1 bis 3 Kohlenstoffatomen, Alkoxyreste mit 1 bis 3 Kohlenstoffatomen, Fluor, Chlor, Brom, Trifluormethylreste oder Dialkylaminoreste mit 2 bis 6 Kohlenstoffatomen sind, $Y^2$ Wasserstoff, ein Alkylrest mit 1 bis 3 Kohlenstoffatomen oder ein Alkoxyrest mit 1 bis 3 Kohlenstoffatomen ist und $Y^1$ und $Y^2$, wenn sie zusammengenommen werden, einen Ethylendioxy- oder Methylendioxyrest bilden; und $R_3$ und $R_4$, wenn sie zusammengenommen werden mit dem Stickstoff, an den sie gebunden sind, einen 4-Phenylpiperazinorest, 4-Alkoxyethoxypiperidinorest, dessen Alkoxyrest 1 bis 3 Kohlenstoffatome hat, oder einen 4-Alkoxycarbonylpiperizinorest, dessen Alkoxyrest 1 bis 3 Kohlenstoffatome hat, bilden, mit dem Vorbehalt, daß dann, wenn $R_1$ ein Alkylrest mit 1 bis 3 Kohlenstoffatomen ist, $R_2$ kein Alkylrest mit 1 bis 8 Kohlenstoffatomen ist,
wenn $X^1$ und $X^2$ jeweils Wasserstoff sind, X ein Aminorest, ein Alkylaminorest mit 1 bis 3 Kohlenstoffatomen, ein Dialkylaminorest mit 2 bis 6 Kohlenstoffatomen oder ein Trifluormethylrest ist,
und wenn $X^1$ H ist und $X^2$ ein Methoxyrest ist oder $X^1$ ein Methoxyrest und $X^2$ Wasserstoff ist, X ein Methoxyrest ist, $R_1$ ein Ethylrest ist, $R_3$ Wasserstoff ist, $R_4$ der Aralkylrest ist, worin W ein Ethylenrest ist und Y, $Y^1$ und $Y^2$ jeweils Wasserstoff sind, dann $R_2$ nicht Ethyl ist, und
wenn $R_1$ ein Alkylrest mit 1 bis 3 Kohlenstoffatomen ist und $R_2$ ein Teil der Formel

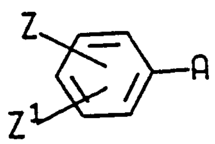

ist, worin A $-CH_2-$ ist, dann Z und Z' nicht beide Wasserstoff sein können,
das umfaßt das Umsetzen einer Verbindung der Formel

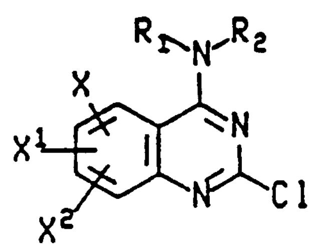

worin $R_1$, $R_2$, X, $X^1$ und $X^2$ wie oben definiert sind, mit einer Verbindung der Formel

$R_3R_4NH$,

worin $R_3$ und $R_4$ wie oben definiert sind, in einem reaktionsinerten Lösungsmittel, das ein Äquivalent eines Amins als Säurefänger umfaßt, bei einer Reaktionstemperatur von 100 bis 200 °C, bis die Reaktion im wesentlichen vollständig ist.

**2.** Verfahren nach Anspruch 1, worin X und $X^1$ jeweils ein Methoxyrest sind, $R_2$ ein Aralkylrest der Formel

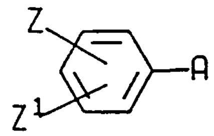

ist, worin Z Wasserstoff ist, $R_3$ Wasserstoff ist und $R_4$ ein Aralkylrest der Formel

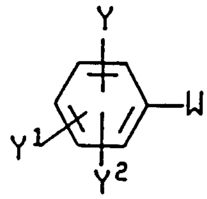

ist, worin W $-(CH_2)_2-$ ist, Y und $Y^1$ jeweils ein Methoxyrest sind und $Y^2$ Wasserstoff ist.

**3.** Verfahren nach Anspruch 2, worin $R_1$ ein Methylrest ist, X ein 6-Methoxyrest ist, $X^1$ din i7-Methoxyrest ist, $X^2$ Wasserstoff ist, $Z^1$ ein 2-Methoxyrest ist, A $-CH_2-$ ist, Y ein 3-Methoyrest ist und $Y^1$ ein 4-Methoxyrest ist.

**4.** Verfahren nach Anspruch 2, worin $R_1$ Wasserstoff ist, X ein 6-Methoxyrest ist, $X^1$ ein 7-Methoxyrest ist, $X^2$ Wasserstoff ist, $Z^1$ Wasserstoff ist, A $-CH_2$ - ist, Y ein 3-Methoxyrest ist und $Y^1$ 4-Methoxyrest ist.

**5.** Verfahren nach Anspruch 2, worin $R_1$ ein Methylrest ist, X ein 6-Methoxyrest ist, $X^1$ ein 7-Methoxyrest ist, $X^2$ Wasserstoff ist, $Z^1$ Wasserstoff ist, A eine chemische Bindung ist, Y ein 3-Methoxyrest ist und $Y^1$ ein 4-Methoxyrest ist.

**6.** Verfahren nach Anspruch 2, worin $R_1$ ein Methylrest ist, X ein 6-Methoxyrest ist, $X^1$ ein 7-Methoxyrest ist, $X^2$ Wasserstoff ist, $Z^1$ ein 4-Fluorrest ist, A $-CH_2-$ ist, Y ein 3-Methoxyrest ist und $Y^1$ ein 4-Methoxyrest ist.

**7.** Verfahren nach Anspruch 1, worin $X^2$ Wasserstoff ist, $R_1$ ein Aralkylrest der Formel

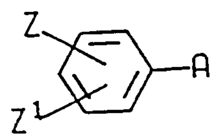

ist, $R_3$ Wasserstoff ist und $R_4$ ein Aralkylrest der Formel

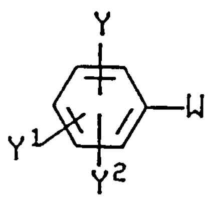

ist, worin W $-(CH_2)_2-$ ist, Y und $Y^1$ jeweils ein Methoxyrest sind und $Y^2$ Wasserstoff ist.

**8.** Verfahren nach Anspruch 7, worin X ein 6-Methoxyrest ist, $X^1$ ein 7-Methoxyrest ist, Z ein 3-Methoxyrest ist, $Z^1$ ein 4-Methoxyrest ist, A eine chemische Bindung ist, Y ein 2-Methoxyrest ist und $Y^1$ 3-Methoxyrest ist.

**9.** Verfahren nach Anspruch 7, worin X ein 6-Methoxyrest ist, $X^1$ ein 7-Methoxyrest ist, Z ein 3-Methoxyrest, $Z^1$ ein 4-Methoxyrest ist, A eine chemische Bindung ist, Y ein 3-Methoxyrest ist und $Y^1$ ein 4-Methoxyrest ist.

**10.** Verfahren nach Anspruch 7, worin X ein 6-Methoxyrest ist, $X^1$ ein 7-Methoxyrest ist, Z ein 2-Methoxyrest ist, Z' ein 3-Methoxyrest ist, A $-CH_2-$ ist, Y ein 3-Methoxyrest ist und $Y^1$ ein 4-Methoxyrest ist.

**Revendications**

**Revendications pour les Etats contractants suivants : AT, BE, CH, DE, DK, FR, GB, IT, LI, LU, NL, SE**

**1.** Composé de formule

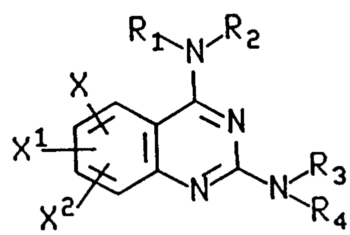

ou un sel d'addition d'acide pharmaceutiquement acceptable de ce composé, formule dans laquelle X est un groupe alkyle ayant un à trois atomes de carbone, un groupe alkoxy ayant un à trois atomes de carbone, un radical chloro, fluoro, un groupe amino, alkylamino ayant un à trois atomes de carbone, un groupe dialkylamino ayant deux à six atomes de carbone ou le groupe trifluorométhyle ; $X^1$ est de l'hydrogène, un groupe alkyle ayant un à trois atomes de carbone, un groupe alkoxy ayant un à trois atomes de carbone, un radical fluoro, chloro ou un groupe dialkylamino ayant deux à six atomes de carbone ; $X^2$ est de l'hydrogène, un groupe alkyle ayant un à trois atomes de carbone ou un groupe alkoxy ayant un à trois atomes de carbone ; X et $X^1$, pris ensemble, représentent un groupe éthylènedioxy ou méthylènedioxy ; $R_1$ est de l'hydrogène ou un groupe alkyle ayant un à trois atomes de carbone ; $R_2$ est un groupe alkyle ayant un à dix atomes de carbone ou un groupement de formule

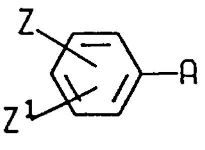

dans laquelle Z et $Z^1$ représentent chacun de l'hydrogène, un groupe alkyle ayant un à trois atomes de carbone, un groupe alkoxy ayant un à trois atomes de carbone, un radical fluoro, chloro, bromo, un groupe trifluorométhyle ou dialkylamino ayant 2 à 6 atomes de carbone, A est une liaison chimique ou un groupe alkylène ayant un à quatre atomes de carbone, et Z et $Z^1$, pris ensemble, forment un groupe éthylènedioxy ou méthylènedioxy ; $R_3$ est de l'hydrogène ou un groupe alkyle ayant un à trois atomes de carbone ; $R_4$ est un groupe aralkyle de formule

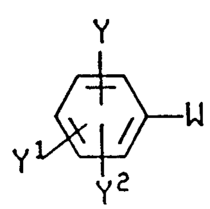

dans laquelle W est un groupe alkylène ayant un à quatre atomes de carbone, Y et $Y^1$ représentent chacun de l'hydrogène, un groupe alkyle ayant un à trois atomes de carbone, un groupe alkoxy ayant un à trois atomes de carbone, un radical fluoro, chloro, bromo, le groupe trifluorométhyle ou un groupe

dialkylamino ayant deux à six atomes de carbone, $Y^2$ est de l'hydrogène, un groupe alkyle ayant un à trois atomes de carbone ou un groupe alkoxy ayant un à trois atomes de carbone et $Y^1$ et $Y^2$, pris ensemble, représentent un groupe éthylènedioxy ou méthylènedioxy ; et $R_3$ et $R_4$, conjointement avec l'atome d'azote auquel ils sont attachés, forment un groupe 4-phénylpipérazino, 4-alkoxyéthoxypipéridino dont le radical alkoxy contient un à trois atomes de carbone ou un groupe 4-alkoxycarbonylpipérizino dont le radical alkoxy contient un à trois atomes de carbone, sous réserve que :

lorsque $R_1$ est un groupe alkyle ayant un à trois atomes de carbone, $R_2$ ne soit pas un groupe alkyle ayant un à huit atomes de carbone ;

lorsque $X^1$ et $X^2$ sont chacun de l'hydrogène, X est un groupe amino, alkylamino ayant un à trois atomes de carbone, dialkylamino ayant deux à six atomes de carbone ou trifluorométhyle ;

lorsque $X^1$ représente H et $X^2$ représente un groupe méthoxy ou bien $X^1$ est un groupe méthoxy et $X^2$ est de l'hydrogène, X est un groupe méthoxy, $R_1$ est un groupe éthyle, $R_3$ est de l'hydrogène, $R_4$ est le groupe aralkyle dans lequel W est de l'éthylène et Y, $Y^1$ et $Y^2$ représentent chacun un atome d'hydrogène, $R_2$ ne soit alors pas un groupe éthyle ; et

lorsque $R_1$ est un groupe alkyle ayant un à trois atomes de carbone et $R_2$ est un groupement de formule

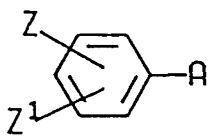

dans laquelle A est un radical $-CH_2-$, Z et $Z^1$ ne puissent pas représenter tous deux de l'hydrogène.

2. Composé suivant la revendication 1, dans lequel X et $X^1$ sont chacun un groupe méthoxy, $R_2$ est un groupe aralkyle de formule

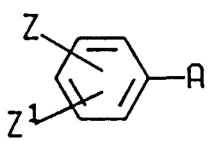

dans laquelle Z est de l'hydrogène, $R_3$ est de l'hydrogène et $R_4$ est un groupe aralkyle de formule

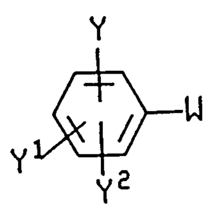

dans laquelle W est un radical $-(CH_2)_2-$, Y et $Y^1$ sont chacun un groupe méthoxy et $Y^2$ est de l'hydrogène.

3. Composé suivant la revendication 2, dans lequel $R_1$ est un groupe méthyle, X est un groupe 6-méthoxy, $X^1$ est un groupe 7-méthoxy, $X^2$ est de l'hydrogène, $Z^1$ est un groupe 2-méthoxy, A est un groupe $-CH_2-$, Y est un groupe 3-méthoxy et $Y^1$ est un groupe 4-méthoxy.

4. Composé suivant la revendication 2, dans lequel $R_1$ est de l'hydrogène, X est un groupe 6-méthoxy, $X^1$ est un groupe 7-méthoxy, $X^2$ est de l'hydrogène, $Z^1$ est de l'hydrogène, A est un groupe $-CH_2-$, Y est un groupe 3-méthoxy et $Y^1$ est un groupe 4-méthoxy.

**5.** Composé suivant la revendication 2, dans lequel $R_1$ est un groupe méthyle, X est un groupe 6-méthoxy, $X^1$ est un groupe 7-méthoxy, $X^2$ est de l'hydrogène, $Z^1$ est de l'hydrogène, A est une liaison chimique, Y est un groupe 3-méthoxy et $Y^1$ est un groupe 4-méthoxy.

**6.** Composé suivant la revendication 2, dans lequel $R_1$ est un groupe méthyle, X est un groupe 6-méthoxy, $X^1$ est 7-méthoxy, $X^2$ est de l'hydrogène, $Z^1$ est un radical 4-fluoro, A est un groupe $-CH_2-$, Y est un groupe 3-méthoxy et $Y^1$ est un groupe 4-méthoxy.

**7.** Composé suivant la revendication 1, dans lequel $X^2$ est de l'hydrogène, $R_1$ est un groupe méthyle, $R_2$ est un groupe aralkyle de formule

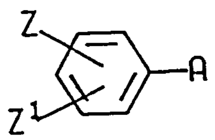

$R_3$ est de l'hydrogène et $R_4$ est un groupe aralkyle de formule

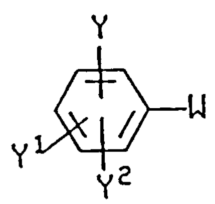

où W est un groupe $-(CH_2)_2-$, Y et $Y^1$ représentent chacun un groupe méthoxy et $Y^2$ est de l'hydrogène.

**8.** Composé suivant la revendication 7, dans lequel X est un groupe 6-méthoxy, $X^1$ est un groupe 7-méthoxy, Z est un groupe 3-méthoxy, $Z^1$ est un groupe 4-méthoxy, A est une liaison chimique, Y est un groupe 2-méthoxy et $Y^1$ est un groupe 3-méthoxy.

**9.** Composé suivant la revendication 7, dans lequel X est un groupe 6-méthoxy, $X^1$ est un groupe 7-méthoxy, Z est un groupe 3-méthoxy, $Z^1$ est un groupe 4-méthoxy, A est une liaison chimique, Y est un groupe 3-méthoxy et $Y^1$ est un groupe 4-méthoxy.

**10.** Composé suivant la revendication 7, dans lequel X est un groupe 6-méthoxy, $X^1$ est un groupe 7-méthoxy, Z est un groupe 2-méthoxy, $Z^1$ est un groupe 3-méthoxy, A est un groupe $-CH_2-$, Y est un groupe 3-méthoxy et $Y^1$ est un groupe 4-méthoxy.

**11.** Utilisation d'un composé suivant la revendication 1 pour la préparation d'un médicament utile pour le traitement ou la prophylaxie de maladies ou de troubles affectant des mammifères sous la médiation d'une P-glycoprotéine.

**12.** Utilisation suivant la revendication 11, dans laquelle le mammifère est un être humain atteint de cancer, et le médicament est administré avant, avec ou après un agent chimiothérapique.

**13.** Composé suivant l'une quelconque des revendications 1 à 10 ou sel d'addition d'acide pharmaceutiquement acceptable de ce composé, destiné à être utilisé comme médicament.

**14.** Composition pharmaceutique destinée à être administrée à un mammifère, qui comprend une quantité inhibitrice de P-glycoprotéine d'un composé suivant la revendication 1, un support acceptable du point de vue pharmaceutique et, à titre facultatif, une quantité à effet anticancéreux d'un agent chimiothérapique.

**15.** Procédé de production d'un composé de formule

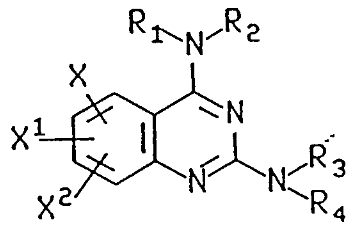

et d'un sel d'addition d'acide pharmaceutiquement accepable de ce composé, formule dans laquelle X est un groupe alkyle ayant un à trois atomes de carbone, un groupe alkoxy ayant un à trois de carbone, un radical chloro, fluoro, un groupe amino, alkylamino ayant un à trois atomes de carbone, un groupe dialkylamino ayant deux à six atomes de carbone ou le groupe trifluorométhyle ; $X^1$ est de l'hydrogène, un groupe alkyle ayant un à trois atomes de carbone, un radical fluoro, chloro ou un groupe dialkylamino ayant deux à six atomes de carbone ; $X^2$ est de l'hydrogène, un groupe alkyle ayant un à trois atomes de carbone ou un groupe alkoxy ayant un à trois atomes de carbone ; X et $X^1$, pris ensemble, représentent un groupe éthylènedioxy ou méthylènedioxy ; $R_1$ est de l'hydrogène ou un groupe alkyle ayant un à trois atomes de carbone ; $R_2$ est un groupe alkyle ayant un à dix atomes de carbone ou un groupement de formule

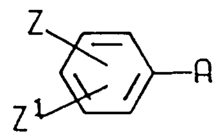

dans laquelle Z et $Z^1$ représentent chacun de l'hydrogène, un groupe alkyle ayant un à trois atomes de carbone, un groupe alkoxy ayant un à trois atomes de carbone, un radical fluoro, chloro, bromo, un groupe trifluorométhyle ou dialkylamino ayant 2 à 6 atomes de carbone, A est une liaison chimique ou un groupe alkylène ayant un à quatre atomes de carbone, et Z et $Z^1$, pris ensemble, forment un groupe éthylènedioxy ou méthylènedioxy ; $R_3$ est de l'hydrogène ou un groupe alkyle ayant un à trois atomes de carbone ; $R_4$ est un groupe aralkyle de formule

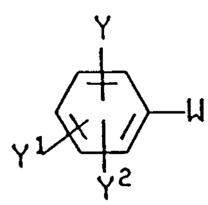

dans laquelle W est un groupe alkylène ayant un à quatre atomes de carbone, Y et $Y^1$ représentent chacun de l'hydrogène, un groupe alkyle ayant un à trois atomes de carbone, un groupe alkoxy ayant un à trois atomes de carbone, un radical fluoro, chloro, bromo, le groupe trifluorométhyle ou un groupe dialkylamino ayant deux à six atomes de carbone, $Y^2$ est de l'hydrogène, un groupe alkyle ayant un à trois atomes de carbone ou un groupe alkoxy ayant un à trois atomes de carbone et $Y^1$ et $Y^2$, pris ensemble, représentent un groupe éthylènedioxy ou méthylènedioxy ; et $R_3$ et $R_4$, conjointement avec l'atome d'azote auquel ils sont attachés, forment un groupe 4-phénylpipérazino, 4-alkoxyéthoxypipéridino dont le radical alkoxy contient un à trois atomes de carbone ou un groupe 4-alkoxycarbonylpipérizino dont le radical alkoxy contient un à trois atomes de carbone, sous réserve que :

lorsque $R_1$ est un groupe alkyle ayant un à trois atomes de carbone, $R_2$ ne soit pas un groupe alkyle ayant un à huit atomes de carbone ;

lorsque $X^1$ et $X^2$ sont chacun de l'hydrogène, X est un groupe amino, alkylamino ayant un à trois atomes de carbone, dialkylamino ayant deux à six atomes de carbone ou trifluorométhyle ; et lorsque

$X^1$ représente H et $X^2$ représente un groupe méthoxy ou bien $X^1$ est un gruope méthoxy et $X^2$ est de l'hydrogène, X est un groupe méthoxy, $R_1$ est un groupe éthyle, $R_3$ est de l'hydrogène, $R_4$ est le groupe aralkyle dans lequel W est de l'éthylène et Y, $Y^1$ et $Y^2$ représentent chacun un atome d'hydrogène, $R_2$ ne soit alors pas un groupe éthyle ;
et lorsque $R_1$ est un groupe alkyle ayant un à trois atomes de carbone et $R_2$ est un groupement de formule

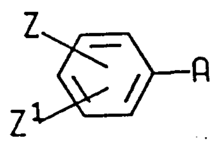

dans laquelle A est un radical $-CH_2-$, Z et $Z^1$ ne puissent pas représenter tous deux de l'hydrogène, qui comprend la réaction d'un composé de formule

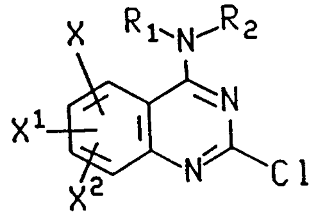

dans laquelle $R_1$, $R_2$, X, $X^1$ et $X^2$ sont tels que définis, avec un composé de formule

$R_3R_4NH$

dans laquelle $R_3$ et $R_4$ sont tels que définis, dans un solvant inerte vis-à-vis de la réaction contenant un équivalent d'un accepteur d'amino-acide à une température de réaction de 100 à 200 °C jusqu'à ce que la réaction soit sensiblement terminée.

**Revendications pour les Etats contractants suivants : ES, GR**

**1.** Procédé de production d'un composé de formule

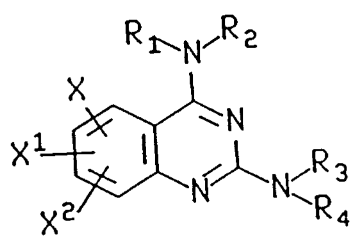

et d'un sel d'addition d'acide pharmaceutiquement accepable de ce composé, formule dans laquelle X est un groupe alkyle ayant un à trois atomes de carbone, un groupe alkoxy ayant un à trois atomes de carbone, un radical chloro, fluoro, un groupe amino, alkylamino ayant un à trois atomes de carbone, un groupe dialkylamino ayant deux à six atomes de carbone ou le groupe trifluorométhyle ; $X^1$ est de l'hydrogène, un groupe alkyle ayant un à trois atomes de carbone, un radical fluoro, chloro ou un groupe dialkylamino ayant deux à six atomes de carbone ; $X^2$ est de l'hydrogène, un groupe alkyle ayant un à trois atomes de carbone ou un groupe alkoxy ayant un à trois atomes de carbone ; X et $X^1$, pris ensemble, représentent un groupe éthylènedioxy ou méthylènedioxy ; $R_1$ est de l'hydrogène ou un groupe alkyle ayant un à trois atomes de carbone ; $R_2$ est un groupe alkyle ayant un à dix atomes de

carbone ou un groupement de formule

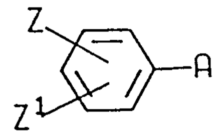

dans laquelle Z et $Z^1$ représentent chacun de l'hydrogène, un groupe alkyle ayant un à trois atomes de carbone, un groupe alkoxy ayant un à trois atomes de carbone, un radical fluoro, chloro, bromo, un groupe trifluorométhyle ou dialkylamino ayant 2 à 6 atomes de carbone, A est une liaison chimique ou un groupe alkylène ayant un à quatre atomes de carbone, et Z et $Z^1$, pris ensemble, forment un groupe éthylènedioxy ou méthylènedioxy ; $R_3$ est de l'hydrogène ou un groupe alkyle ayant un à trois atomes de carbone ; $R_4$ est un groupe aralkyle de formule

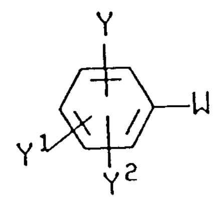

dans laquelle W est un groupe alkylène ayant un à quatre atomes de carbone, Y et $Y^1$ représentent chacun de l'hydrogène, un groupe alkyle ayant un à trois atomes de carbone, un groupe alkoxy ayant un à trois atomes de carbone, un radical fluoro, chloro, bromo, le groupe trifluorométhyle ou un groupe dialkylamino ayant deux à six atomes de carbone, $Y^2$ est de l'hydrogène, un groupe alkyle ayant un à trois atomes de carbone ou un groupe alkoxy ayant un à trois atomes de carbone et $Y^1$ et $Y^2$, pris ensemble, représentent un groupe éthylènedioxy ou méthylènedioxy ; et $R_3$ et $R_4$, conjointement avec l'atome d'azote auquel ils sont attachés, forment un groupe 4-phénylpipérazino, 4-alkoxyéthoxypipéridino dont le radical alkoxy contient un à trois atomes de carbone ou un groupe 4-alkoxycarbonylpipérizino dont le radical alkoxy contient un à trois atomes de carbone, sous réserve que :

lorsque $R_1$ est un groupe alkyle ayant un à trois atomes de carbone ,$R_2$ ne soit pas un groupe alkyle ayant un à huit atomes de carbone ;

lorsque $X^1$ et $X^2$ sont chacun de l'hydrogène, X est un groupe amino, alkylamino ayant un à trois atomes de carbone, dialkylamino ayant deux à six atomes de carbone ou trifluorométhyle ;

et lorsque $X^1$ représente H et $X^2$ représente un groupe méthoxy ou bien $X^1$ est un groupe méthoxy et $X^2$ est de l'hydrogène, X est un groupe méthoxy, $R_1$ est un groupe éthyle, $R_3$ est de l'hydrogène, $R_4$ est le groupe aralkyle dans lequel W est de l'éthylène et Y, $Y^1$ et $Y^2$ représentent chacun un atome d'hydrogène, $R_2$ ne soit alors pas un groupe éthyle ; et

lorsque $R_1$ est un groupe alkyle ayant un à trois atomes de carbone et $R_2$ est un groupement de formule

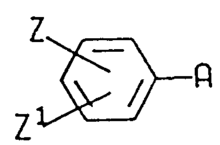

dans laquelle A est un radical $-CH_2-$, Z et $Z^1$ ne puissent pas représenter tous deux de l'hydrogène, qui comprend la réaction d'un composé de formule

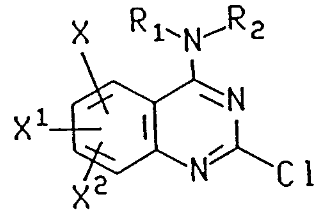

dans laquelle $R_1$, $R_2$, X, $X^1$ et $X^2$ sont tels que définis, avec un composé de formule

$R_3R_4NH$

dans laquelle $R_3$ et $R_4$ sont tels que définis, dans un solvant inerte vis-à-vis de la réaction contenant un équivalent d'un accepteur d'amino-acide à une température de réaction de 100 à 200°C jusqu'à ce que la réaction soit sensiblement terminée.

2. Procédé suivant la revendication 1, dans lequel X et $X^1$ représentent chacun un groupe méthoxy, $R_2$ est un groupe aralkyle de formule

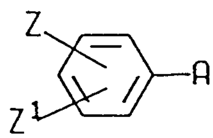

dans laquelle Z est de l'hydrogène, $R_3$ est de l'hydrogène et $R_4$ est un groupe aralkyle de formule

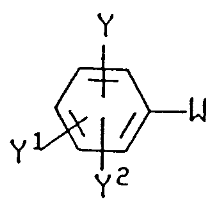

dans laquelle W est un groupe - $(CH_2)_2$-, Y est $Y^1$ représentent chacun un groupe méthoxy et $Y^2$ est de l'hydrogène.

3. Procédé suivant la revendication 2, dans lequel $R_1$ est un groupe méthyle, X est un groupe 6-méthoxy, $X^1$ est un groupe 7-méthoxy, $X^2$ est de l'hydrogène, $Z^1$ est un groupe 2-méthoxy, A est un groupe $-CH_2-$, Y est un groupe 3-méthoxy et $Y^1$ est un groupe 4-méthoxy.

4. Procédé suivant la revendication 2, dans lequel $R_1$ est de l'hydrogène, X est un groupe 6-méthoxy, $X^1$ est un groupe 7-méthoxy, $X^2$ est de l'hydrogène, $Z^1$ est de l'hydrogène, A est un groupe $-CH_2-$, Y est un groupe 3-méthoxy et $Y^1$ est un groupe 4-méthoxy.

5. Procédé suivant la revendication 2, dans lequel $R_1$ est un groupe méthyle, X est un groupe 6-méthoxy, $X^1$ est un groupe 7-méthoxy, $X^2$ est de l'hydrogène, $Z^1$ est de l'hydrogène, A est une liaison chimique, Y est un groupe 3-méthoxy et $Y^1$ est un groupe 4-méthoxy.

6. Procédé suivant la revendication 2, dans lequel $R_1$ est un groupe méthyle, X est un groupe 6-méthoxy, $X^1$ est un groupe 7-méthoxy, $X^2$ est de l'hydrogène, $Z^1$ est un radical 4-fluoro, A est un groupe $-CH_2-$, Y est un groupe 3-méthoxy et $Y^1$ est un groupe 4-méthoxy.

7. Procédé suivant la revendication 1, dans lequel $X^2$ est de l'hydrogène, $R_1$ est un groupe aralkyle de formule

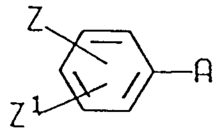

$R_3$ est de l'hydrogène et $R_4$ est un groupe aralkyle de formule

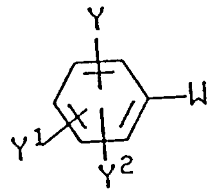

où W est un groupe $-(CH_2)_2-$, Y et $Y^1$ sont chacun un groupe méthoxy et $Y^2$ est de l'hydrogène.

**8.** Procédé suivant la revendication 7, dans lequel X est un groupe 6-méthoxy, $X^1$ est un groupe 7-méthoxy, Z est un groupe 3-méthoxy, $Z^1$ est un groupe 4-méthoxy, A est une liaison chimique, Y est un groupe 2-méthoxy et $Y^1$ est un groupe 3-méthoxy.

**9.** Procédé suivant la revendication 7, dans lequel X est un groupe 6-méthoxy, $X^1$ est un groupe 7-méthoxy, Z est un groupe 3-méthoxy, $Z^1$ est un groupe 4-méthoxy, A est une liaison chimique, Y est un groupe 3-méthoxy et $Y^1$ est un groupe 4-méthoxy.

**10.** Procédé suivant la revendication 7, dans lequel X est un groupe 6-méthoxy, $X^1$ est un groupe 7-méthoxy, Z est un groupe 2-méthoxy, $Z^1$ est un groupe 3-méthoxy, A est un groupe $-CH_2-$, Y est un groupe 3-méthoxy et $Y^1$ est un groupe 4-méthoxy.